# EUROPEAN PATENT APPLICATION

(11) **EP 1 236 476 A1**
(43) Date of publication of application: **04.09.2002**
(21) Application number: 00979962.8
(22) Date of filing: 07.12.2000
(51) Int. Cl.: A61K 45/08, A61K 9/08, A61K 9/10, A61K 47/10, A61K 47/12, A61K 47/44, C07D 309/14, C07D 405/12, C07D 407/12, C07D 295/135, C07D 313/08, C07D 409/12, A61K 31/351, A61K 31/55, A61K 31/445, A61K 31/335, A61K 31/18, A61P 43/00, A61P 31/18

(54) **MEDICINAL COMPOSITIONS FOR ORAL USE**

(30) Priority: 10.12.1999 JP 35179899; 16.02.2000 JP 2000043600
(71) Applicant: Takeda Chemical Industries, Ltd., Osaka-shi, Osaka 541-0045 (JP)
(72) Inventor: AKIYAMA, Yohko, Ohmihachiman-shi Shiga 523-0891 (JP); NAGAHARA, Naoki, 752 42 Uppsala (SE); MATSUMOTO, Yukihiro, Kobe-shi Hyogo 651-0056 (JP)
(74) Representative: Lewin, John Harvey
(86) International application number: JP0008648
(87) International publication number: WO01041808

(57) **Abstract**

Medicinal compositions for oral use to be used as preparations for oral administration showing little scatter in the concentration of a compound, which is insoluble or slightly soluble in water and shows a CCR5 antagonism, or its salt in blood, having very high oral absorbability and being useful in preventing and treating various HIV infections in humans, characterized in that the above-described compound or its salt is dissolved or dispersed in a carrier containing at least one constituent selected from among amphipathic substances, alcohols and lipids.

## Description

### FIELD OF THE INVENTION

The present invention relates to a pharmaceutical composition for oral administration, which comprises a compound having CCR5 antagonism or salts thereof.

### BACKGROUND ART

HIV (human immunodeficiency virus) is replicated and multiplied by repeating a life cycle consisting of adsorption onto host cells, entry/uncoating, reverse transcription of viral genes and integration thereof in host genes, transcription/replication of the viral genes, synthesis and modification of proteins, and formation and release of particles. An agent capable of inhibiting any stage in this cycle can serve as an inhibitor of growth of HIV and AIDS viruses.

CD4 has been known as a receptor for entry of HIV into target cells, and recently CCR5 was found as macrophage-targeting HIV second receptor, and G protein-conjugated chemokine receptor of 7 times transmembrane type called CXCR4 was found as T cell-targeting second receptor, and it is estimated that these chemokine receptors play an essential role in establishment and spread of infection with HIV. In fact, there is also a report showing that even upon repeated exposure, humans resistant to infection with HIV had a homo deletion mutation on the CCR5 gene. Accordingly, it is expected that a CCR5 antagonist can serve as a new anti-HIV drug.

It is desired that such anti-HIV drug is developed in the form of a preparation capable of oral administration, and whether such a preparation for oral administration can be developed or not depends on the bioavailability of the drug. Factors affecting bioavailability include drug solubility, permeability through digestive tract membrane, or the first pass effect, but many compounds having CCR5 antagonism or salts thereof are hydrophobic thus having the fault of low bioavailability upon oral administration. Accordingly, improvements in the oral absorbability of compounds having CCR5 antagonism or salts thereof are also important for stably exhibiting the efficacy thereof as CCR5 antagonists.

As described above, the development of anti-HIV drug as a preparation for oral administration is most preferable from the viewpoint of compliance, and the therapeutic effect of the preparation poor in bioavailability can be varied, so the development of a preparation for oral administration with improvements in bioavailability, including a compound having CCR5 antagonism or a salt thereof, is desired at present.

### DISCLOSURE OF THE INVENTION

In view of these circumstances, the present inventors examined formulations of preparations for oral administration having higher bioavailability, and as a result they found that the oral absorbability of the compounds having CCR5 antagonism or salts thereof can be improved by finely dispersing or dissolving the compounds having CCR5 antagonism or salts thereof in a specific substance, and on the basis of this knowledge, the present invention was completed.

That is, the present invention relates to:
(1) A pharmaceutical composition for oral use, which comprises a water-insoluble or slightly soluble compound having CCR5 antagonism or a salt thereof dissolved or dispersed in a carrier including at least one component selected from amphipathic substances, alcohols and lipids;
(2) A pharmaceutical composition for oral use, which comprises a water-insoluble or slightly soluble compound having CCR5 antagonism or a salt thereof dissolved in a carrier including at least one component selected from amphipathic substances, alcohols and lipids;
(3) A pharmaceutical composition for oral use, which comprises a water-insoluble or slightly soluble compound having CCR5 antagonism or a salt thereof dispersed in a carrier including at least one component selected from amphipathic substances, alcohols and lipids;
(4) The composition according to any one of the above-mentioned (1) to (3), which is a liquid at ordinary temperature;
(5) The composition according to any one of the above-mentioned (1) to (3), which is a semisolid (for example, in the form of a paste) at ordinary temperature;
(6) The composition according to any one of the above-mentioned (1) to (3), which is a solid at ordinary temperature;
(7) The composition according to any one of the above-mentioned (1) to (3), wherein the carrier includes an amphipathic substance;
(8) The composition according to any one of the above-mentioned (1) to (3), wherein the carrier includes at least one amphipathic substance having high HLB and at least one amphipathic substance having low HLB respectively; the composition according to the above-mentioned (8), which further includes at least one alcohol or at least one lipid;
(9) The composition according to any one of the above-mentioned (1) to (3), wherein the carrier includes an alcohol;
(10) The composition according to any one of the above-mentioned (1) to (3), wherein the carrier includes a lipid;
(11) The composition according to any one of the above-mentioned (1) to (3), wherein the carrier includes at least one alcohol and at least one lipid respectively.
(12) The composition according to any one of the above-mentioned (1) to (3), wherein the carrier includes at least one alcohol and at least one amphipathic substance respectively;
(13) The composition according to any one of the above-mentioned (1) to (3), wherein the carrier includes at least one amphipathic substance and at least one lipid respectively; and the composition according to the above-mentioned (13), which further includes at least one alcohol;
(14) The composition according to any one of the above-mentioned (1) to (3), wherein the carrier includes an absorption-promoting additive;
(15) The composition according to the above-mentioned (14), wherein the absorption-promoting additive is an alkali metal salt or alkaline earth metal salt with an organic acid;
(16) The composition according to the above-mentioned (15), wherein the organic acid is salicylic acid, deoxycholic acid, myristic acid, dodecylsulfuric acid, decylsulfuric acid, tetradecylsulfuric acid or lauric acid;
(17) The composition according to the above-mentioned (14), wherein the absorption-promoting additives is sodium salicylate, sodium deoxycholate, sodium myristate, sodium dodecylsulfate, sodium decylsulfate, sodium tetradecylsulfate or sodium laurate;
(18) The composition according to any one of the above-mentioned (1) to (3), wherein the amphipathic substance is a self-emulsifiable surfactant;
(19) The composition according to the above-mentioned (7), wherein the amphipathic substance is a combination of one or more amphipathic substances selected from saturated polyglycolized glycerides, unsaturated polyglycolized glycerides and D-α-tocopheryl polyethylene glycol 1000 succinate;
(20) The composition according to the above-mentioned (19), which further includes an alkali metal salt or alkaline earth metal salt with an organic acid;
(21) The composition according to the above-mentioned (9), wherein the alcohol is propylene glycol, lauryl alcohol or diethylene glycol monoethyl ether;
(22) A pharmaceutical composition for oral use wherein an emulsion is formed by dissolving or dispersing a water-insoluble or slightly soluble compound having CCR5 antagonism or a salt thereof in a carrier including at least one component selected from amphipathic substances, alcohols and lipids, followed by adding water thereto;
(23) The composition according to any one of the above-mentioned (1) to (3), wherein the compound having CCR5 antagonism or a salt thereof is a compound represented by formula (I);

   **R**^{**1**}**―X**^{**1**}**―W―X**^{**2**}**―Z**^{**1**}**―Z**^{**2**}**―R**^{**2**}

   wherein R¹ represents an optionally substituted 5- to 6-membered ring, X¹ represents a bond or a divalent group having 1 to 4 atoms in the linear chain moiety thereof, W is a divalent group represented by the following formula; 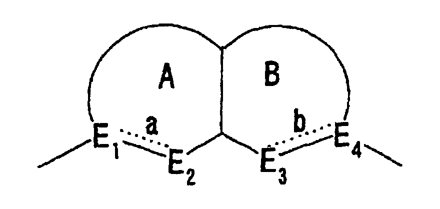 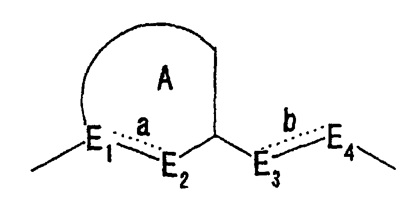 or 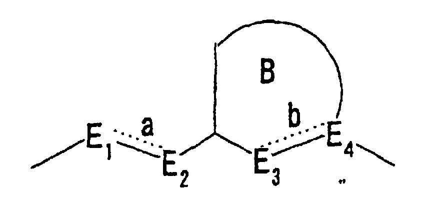 wherein rings A and B each represent an optionally substituted 5- to 7-membered ring, E₁ and E₄ each represent an optionally substituted carbon atom or an optionally substituted nitrogen atom, E₂ and E₃ each represent an optionally substituted carbon atom, an optionally substituted nitrogen atom, an optionally oxidized sulfur atom or an oxygen atom, and break lines a and b each represent a single or double bond; X² represents a divalent group having 1 to 4 atoms in the linear-chain moiety thereof, Z¹ represents a bond or a divalent cyclic group, Z² represents a bond or a divalent group having 1 to 4 carbon atoms in the linear-chain moiety thereof, R² represents (1) optionally substituted amino group whose nitrogen atom may be converted into quaternary ammonium or oxide, (2) optionally substituted nitrogen-containing heterocyclic group whose nitrogen atom may be converted into quaternary ammonium or oxide and which may contain a sulfur atom or oxygen atom as a ring constituent atom, (3) group bound via a sulfur atom, (4) group represented by the following formula: 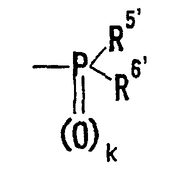 wherein k is 0 or 1, and when k is 0, the phosphorus atom may form a phosphonium salt, and R⁵' and R⁶' each represent an optionally substituted hydrocarbon group, an optionally substituted hydroxy group or an optionally substituted amino group, and R⁵' and R⁶' may be bound to each other to form a cyclic ring with their adjacent phosphorus atom, (5) optionally substituted amidino group, or (6) optionally substituted guanidino group, or a salt thereof;
(24) The composition according to any one of the above-mentioned (1) to (3), wherein the compound having CCR5 antagonism or a salt thereof is a compound represented by formula (Ia): 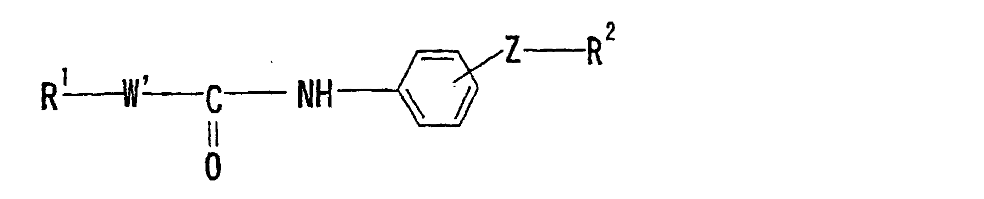 wherein R¹ has the same meaning as defined in the above-mentioned (23), and W' is a divalent group represented by: 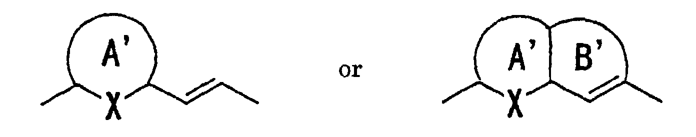 wherein ring A' represents an optionally substituted 5- to 6-membered aromatic ring, X represents an optionally substituted carbon atom, an optionally substituted nitrogen atom, a sulfur atom or an oxygen atom, and ring B' represents an optionally substituted 5- to 7-membered ring; Z represents a bond or a divalent group having 1 to 4 carbon atoms in the linear-chain moiety thereof, and R² has the same meaning as defined in the above-mentioned (23), or a salt thereof;
(25) The composition according to any one of the above-mentioned (1) to (3), wherein the compound having CCR5 antagonism or a salt thereof is N,N-dimethyl-N-(4-(((2-(4-methylphenyl)-6,7-dihydro-5H-benzocyclohepten-8-yl) carbonyl) amino) benzyl)-N-(4-tetrahydropyranyl) ammonium chloride.
(26) The composition according to the above-mentioned (23), wherein R¹ is a group formed by removing one hydrogen atom from optionally substituted benzene, furan, thiophene, pyridine, cyclopentane, cyclohexane, pyrrolidine, piperidine, piperazine, morpholine, thiomorpholine or tetrahydropyran;
(27) The composition according to the above-mentioned (23), wherein R¹ is optionally substituted phenyl;
(28) The composition according to the above-mentioned (23), wherein X¹ is a bond, -(CH₂)ₐ,- [a' is an integer of 1 to 4], -(CH₂)_{b},-X³- [b' is an integer of 0 to 3, and X³ is an optionally substituted imino group, a carbonyl group, an oxygen atom or an optionally oxidized sulfur atom], -CH=CH-, -C≡C-, -CO-NH- or -SO₂-NH-;
(29) The composition according to the above-mentioned (23), wherein X¹ is a bond;
(30) The composition according to the above-mentioned (23), wherein X¹ is -(CH₂)_{b},-X³- [b' is an integer of 0 to 3, and X³ is an optionally substituted imino group, a carbonyl group, an oxygen atom or an optionally oxidized sulfur atom];
(31) The composition according to the above-mentioned (23), wherein ring A represents optionally substituted furan, thiophene, pyrrole, pyridine, pyran or benzene;
(32) The composition according to the above-mentioned (23), wherein ring A is optionally substituted benzene;
(33) The composition according to the above-mentioned (23), wherein ring B is a 5- to 7-membered ring optionally substituted with a substituent group at an arbitrary replaceable position, represented by the formula: 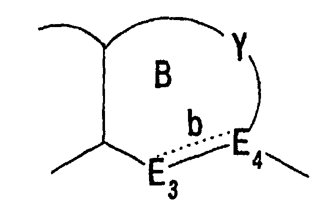 wherein E₃ represents an optionally substituted carbon atom or an optionally substituted nitrogen atom, E₄ represents an optionally substituted carbon atom or a nitrogen atom, break line b represents a single or double bond, Y represents -Y'-(CH₂)m'- (Y' is -S(O)ₘ- (m is an integer of 0 to 2), -O-, -NH- or -CH₂-, and m' is an integer of 0 to 2), -CH=, -CH=CH- or -N=CH-;
(34) The composition according to the above-mentioned (33), wherein Y is -Y'-(CH₂)₂- whereupon Y' is -S(O)ₘ- (m is an integer of 0 to 2), -O-, -NH- or -CH₂-;
(35) The composition according to the above-mentioned (23), wherein E₃ represents an optionally substituted carbon atom, E₄ represents an optionally substituted carbon atom, and break line b is a double bond;
(36) The composition according to the above-mentioned (23), wherein X² represents - (CH₂)ₐ,- [a' is an integer of 1 to 4], -(CH₂)_{b},-X³- [b' is an integer of 0 to 3, X³ represents an optionally substituted imino group, a carbonyl group, an oxygen atom or an optionally oxidized sulfur atom], -CH=CH-, -C≡C-, -CO-NH- or -SO₂-NH-;
(37) The composition according to the above-mentioned (23), wherein X² is -CO-NH-;
(38) The composition according to the above-mentioned (23), wherein Z¹ is (1) a bond or (2) a divalent cyclic group formed by removing 2 hydrogen atoms from optionally substituted benzene, furan, thiophene, pyridine, cyclopentane, cyclohexane, pyrrolidine, piperidine, piperazine, morpholine, thiomorpholine or tetrahydropyran;
(39) The composition according to the above-mentioned (23), wherein Z¹ is (1) a bond or (2) a divalent cyclic group formed by removing 2 hydrogen atoms from optionally substituted benzene, cyclohexane or piperidine;
(40) The composition according to the above-mentioned (23), wherein Z¹ is optionally substituted phenylene;
(41) The composition according to the above-mentioned (23), wherein Z² is a bond or optionally substituted C₁₋₃ alkylene;
(42) The composition according to the above-mentioned (23), wherein Z² is a divalent group having a skeleton represented by -Z'-(CH₂)n- [Z' represents -CH(OH)-, -C(O)-or -CH₂-, n is an integer of 0 to 2], and optionally having a substituent group on an arbitrary methylene group;
(43) The composition according to the above-mentioned (23), wherein Z² is a bond or methylene;
(44) The composition according to the above-mentioned (23), wherein Z¹ is a 6-membered divalent cyclic group, and the position at which Z² is substituted is a p-position relative to X².
(45) The composition according to the above-mentioned (23), wherein R² represents (1) optionally substituted amino group whose nitrogen atom may be converted into quaternary ammonium or oxide, (2) optionally substituted nitrogen-containing heterocyclic group whose nitrogen atom may be converted into quaternary ammonium or oxide and which may contain a sulfur atom or oxygen atom as a ring constituent atom, (3) optionally substituted amidino group, or (4) optionally substituted guanidino group;
(46) The composition according to the above-mentioned (23), wherein R² is an optionally substituted amino group;
(47) The composition according to the above-mentioned (23), wherein R² is an optionally substituted amidino group or an optionally substituted guanidino group;
(48) The composition according to the above-mentioned (23), wherein R² is an amino group which is converted into quaternary ammonium;
(49) The composition according to any one of the above-mentioned (1) to (3), wherein the compound having CCR5 antagonism or a salt thereof is a salt with an organic acid;
(50) The composition according to the above-mentioned (49), wherein the organic acid is salicylic acid, deoxycholic acid, myristic acid, dodecylsulfuric acid, decylsulfuric acid, tetradecylsulfuric acid or lauric acid;
(51) The composition according to any one of the above-mentioned (1) to (3), which further includes a combination of protease inhibitors and/or reverse transcriptase inhibitors;
(52) Capsules including the composition according to any one of the above-mentioned (1) to (3);
(53) A process for producing the composition according to any one of the above-mentioned (1) to (3), which includes dissolving or dispersing a water-insoluble or slightly soluble compound having CCR5 antagonism or a salt thereof in a carrier melted at a temperature higher than the melting point thereof (preferably a temperature higher by at least 10 °C than the melting point, more preferably a temperature higher by at least 20 °C than the melting point) followed by cooling thereof;
(54) The composition according to any one of the above-mentioned (1) to (3), which includes water;
(55) The composition according to any one of the above-mentioned (1) to (3) in the form of an emulsion prepared by adding water;
(56) The composition according to any one of the above-mentioned (1) to (3), which is prepared from a dispersion of a water-insoluble or slightly soluble compound having CCR5 antagonism or a salt thereof in water or an aqueous solution thereof prepared by heating and a carrier including at least one component selected from amphipathic substances, alcohols and lipids;
(57) The composition according to any one of the above-mentioned (1) to (3), which is an anti-HIV drug;
(58) A salt of a water-insoluble or slightly soluble compound having CCR5 antagonism formed with salicylic acid, deoxycholic acid, myristic acid, dodecylsulfuric acid, decylsulfuric acid, tetradecylsulfuric acid or lauric acid; and
(59) A salt comprising N,N-dimethyl-N-(4-(((2-(4-methylphenyl)-6,7-dihydro-5H-benzocyclohepten-8-yl) carbonyl) amino) benzyl)-N-(4-tetrahydropyranyl) ammonium and an anion derived from salicylic acid, deoxycholic acid, myristic acid, dodecylsulfuric acid, decylsulfuric acid, tetradecylsulfuric acid or lauric acid.

The "compound having CCR5 antagonism" used in the present invention may be any compounds sparingly soluble in water and inhibiting or regulating entry of macrophage-targeting HIV into target cells via CC ckemokine 5-receptor (CCR5) (for example, compounds having antagonism in binding of natural ligand RANTES to CCR5), and specifically, the compounds having CCR5 antagonism or salts thereof which are sparingly soluble in water include, but are not limited to, those described in Japanese Patent Application No. 127724 (1999) (PCT/JP00/02825), WO99/32100, WO99/32468, PCT/JP99/04454 (WO00/10965), Japanese Patent Application No. 170212 (1999) (WO00/37455), Japanese Patent Application No. 170345 (1999) (PCT/JP00/03879), Proc. Natl. Acad. Sci., USA, 96, 5698-5703 (1999), NSC-651016 (J. Med. Chem., 41, 2184 (1998)), WO98/25604, WO98/25605, WO98/25617, WO98/27815, WO98/30218, WO98/31364, WO99/1127, WO99/4797, WO99/9984 and WO99/14378 [preferable examples include N,N-dimethyl-N-(4-(((2-(4-methylphenyl)-6,7-dihydro-5H-benzocyclohepten-8-yl)carbonyl)amino)benzyl)-N-(4-tetrahydropyranyl)ammonium chloride, etc.].

In particular, the compound or a salt thereof is a compound represented by formula (I):

**R**^{**1**}**―X**^{**1**}**―W―X**^{**2**}**―Z**^{**1**}**―Z**^{**2**}**―R**^{**2**}

wherein R¹ represents an optionally substituted 5- to 6-membered ring, X¹ represents a bond or a divalent group having 1 to 4 atoms in the linear chain moiety thereof, W is a divalent group represented by the following formula: 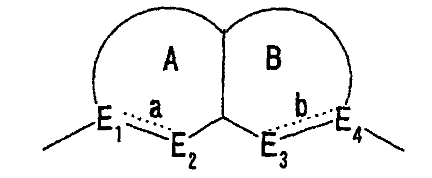 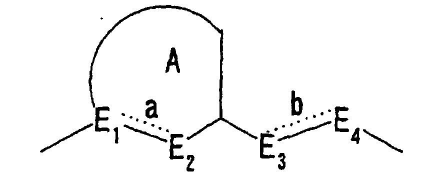 or 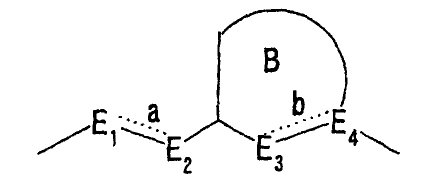 wherein rings A and B each represent an optionally substituted 5- to 7-membered ring, E₁ and E₄ each represent an optionally substituted carbon atom or an optionally substituted nitrogen atom, E₂ and E₃ each represent an optionally substituted carbon atom, an optionally substituted nitrogen atom, an optionally oxidized sulfur atom or an oxygen atom, and broken lines a and b each represent a single or double bond, X² represents a divalent group having 1 to 4 atoms in the linear-chain moiety thereof, Z¹ represents a bond or a divalent cyclic group, Z² represents a bond or a divalent group having 1 to 4 carbon atoms in the linear-chain moiety thereof; R² represents (1) optionally substituted amino group whose nitrogen atom may be converted into quaternary ammonium or oxide, (2) optionally substituted nitrogen-containing heterocyclic group whose nitrogen atom may be converted into quaternary ammonium or oxide and which may contain a sulfur atom or oxygen atom as a ring constituent atom, (3) group bound via a sulfur atom, (4) group represented by the following formula: 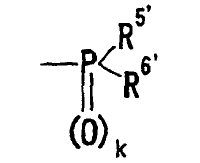 wherein k is 0 or 1, and when k is 0, the phosphorus atom may form a phosphonium salt, and R⁵' and R⁶' each represent an optionally substituted hydrocarbon group, an optionally substituted hydroxy group or an optionally substituted amino group, and R⁵' and R⁶' may be bound to each other to form a cyclic ring with their adjacent phosphorus atom, (5) optionally substituted amidino group, or (6) optionally substituted guanidino group, or a salt thereof.

The compound or a salt thereof is particularly preferably a compound represented by formula (Ia): 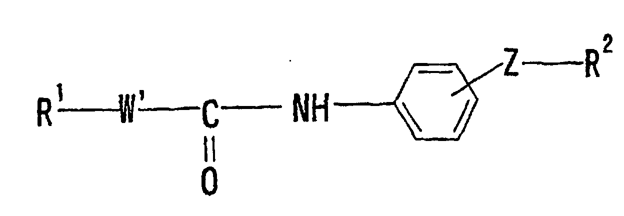 wherein R¹ has the same meaning as defined above, and W' is a divalent group represented by: 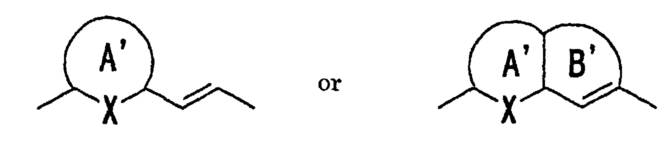 wherein ring A' represents an optionally substituted 5- to 6-membered aromatic ring, X represents an optionally substituted carbon atom, an optionally substituted nitrogen atom, a sulfur atom or an oxygen atom, and ring B' represents an optionally substituted 5- to 7-membered ring, Z represents a bond or a divalent group having 1 to 4 carbon atoms in the linear-chain moiety thereof, and R² has the same meaning as defined above [preferably, (1) optionally substituted amino group whose nitrogen atom may be converted into quaternary ammonium, (2) optionally substituted nitrogen-containing heterocyclic group whose nitrogen atom may be converted into quaternary ammonium and which may contain a sulfur atom or oxygen atom as a ring constituent atom, (3) group bound via a sulfur atom, or (4) group represented by the following formula: 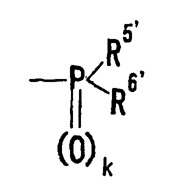 wherein k is 0 or 1, and when k is 0, the phosphorus atom may form a phosphonium salt, and R⁵' and R⁶' each represent an optionally substituted hydrocarbon group, an optionally substituted hydroxy group or an optionally substituted amino group, and R⁵' and R⁶' may be bound to each other to form a cyclic ring with their adjacent phosphorus atom, or a salt thereof.

In the "optionally substituted 5- to 6-membered cyclic group" represented by R¹ in the formula (I) above, the "5-to 6-membered cyclic group" includes groups formed by removing one hydrogen atom from 6-membered aromatic hydrocarbons such as benzene, 5- to 6-membered aliphatic hydrocarbons such as cyclopentane, cyclohexane, cyclopentene, cyclohexene, cyclopentane diene and cyclohexane diene, 5- to 6-membered aromatic heterocyclic rings containing one or two kinds of 1 to 4 heteroatoms selected from a nitrogen atom, a sulfur atom and an oxygen atom, such as furan, thiophene, pyrrole, imidazole, pyrazol, thiazole, oxazole, isothiazole, isoxazole, tetrazole, pyridine, pyrazine, pyrimidine, pyridazine and triazole, or 5- to 6-membered non-aromatic heterocyclic rings containing one or two kinds of 1 to 4 heteroatoms selected from a nitrogen atom, a sulfur atom and an oxygen atom, such as tetrahydrofuran, tetrahydrothiophene, dithiolan, oxathiolan, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran and tetrahydrothiopyran, among which the "5- to 6-membered ring" is preferably benzene, furan, thiophene, pyridine, cyclopentane, cyclohexane, pyrrolidine, piperidine, piperazine, morpholine, thiomorpholine or tetrahydropyran (preferably 6-membered ring), particularly preferably benzene.

The "substituent groups" possessed by the "5- to 6-membered ring" in the "optionally substituted 5- to 6-membered cyclic group" represented by R¹ include for example a halogen atom, nitro and cyano, an optionally substituted alkyl, an optionally substituted cycloalkyl, an optionally substituted hydroxyl group, an optionally substituted thiol group (whose sulfur atom may be oxidized or may form an optionally substituted sulfinyl group or an optionally substituted sulfonyl group), an optionally substituted amino group, an optionally substituted acyl, an optionally esterified carboxyl group and an optionally substituted aromatic group.

The halogen as a substituent group on R¹ includes e.g. fluorine, chlorine, bromine and iodine, among which fluorine and chlorine are particularly preferable.

The alkyl in the optionally substituted alkyl as a substituent group on R¹ includes linear or branched alkyl containing 1 to 10 carbon atoms, for example C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower (C₁₋₆) alkyl. The substituent group on the optionally substituted alkyl includes halogens (e.g., fluorine, chlorine, bromine, iodine etc.), nitro, cyano, hydroxyl group, thiol group, amino group, carboxyl group, optionally halogenated C₁₋₄ alkoxy (e.g., methoxy, ethoxy, propoxy, butoxy, trifluoromethoxy, trifluoroethoxy and the like), formyl, C₂₋₄ alkanoyl (e.g., acetyl, propionyl and the like) and C₁₋₄ alkyl sulfonyl (e.g., methane sulfonyl, ethane sulfonyl and the like), and the number of such substituent groups is preferably 1 to 3.

The cycloalkyl in the optionally substituted cycloalkyl as a substituent group on R¹ includes for example C₃₋₇ cycloalkyl such as cyclopropyl, cyclobutyl, cyclopentyl, cyclohexyl and cycloheptyl. The substituent group on the optionally substituted cycloalkyl includes halogens (e.g., fluorine, chlorine, bromine, iodine and the like), nitro, cyano, hydroxyl group, thiol group, amino group, carboxyl group, optionally halogenated C₁₋₄ alkyl (e.g., trifluoromethyl, methyl, ethyl and the like), optionally halogenated C₁₋₄ alkoxy (e.g., methoxy, ethoxy, propoxy, butoxy, trifluoromethoxy, trifluoroethoxy and the like), formyl, C₂₋₄ alkanoyl (e.g., acetyl, propionyl and the like), C₁₋₄ alkyl sulfonyl (e.g., methane sulfonyl, ethane sulfonyl and the like) etc., and the number of such substituent groups is preferably 1 to 3.

The substituent group on the optionally substituted hydroxyl group as a substituent group on R¹ includes:
(1) optionally substituted alkyl (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower (C₁₋₆) alkyl);
(2) optionally substituted cycloalkyl (for example, C₃₋₇ cycloalkyl such as cyclopropyl, cyclobutyl, cyclopentyl, cyclohexyl and cycloheptyl);
(3) optionally substituted alkenyl (for example, alkenyl containing 2 to 10 carbon atoms such as allyl, crotyl, 2-pentenyl and 3-hexenyl, preferably lower (C₂₋₆) alkenyl);
(4) optionally substituted cycloalkenyl (for example, cycloalkenyl containing 3 to 7 carbon atoms such as 2-cyclopentenyl, 2-cyclohexenyl, 2-cyclopentenyl methyl and 2-cyclohexenyl methyl);
(5) optionally substituted aralkyl (for example, phenyl-C₁₋₄ alkyl (e.g., benzyl, phenethyl etc.));
(6) formyl or optionally substituted acyl (for example, alkanoyl containing 2 to 4 carbon atoms (for example, acetyl, propionyl, butyryl, isobutyryl and the like), alkyl sulfonyl containing 1 to 4 carbon atoms (e.g., methane sulfonyl, ethane sulfonyl and the like) etc.; and
(7) optionally substituted aryl (for example, phenyl, naphthyl and the like).

The substituent groups which may be possessed by (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl, and (7) optionally substituted aryl described above include halogens (e.g., fluorine, chlorine, bromine, iodine and the like), nitro, cyano, hydroxyl group, thiol group, amino group, carboxyl group, optionally halogenated C₁₋₄ alkyl (e.g., trifluoromethyl, methyl, ethyl and the like), optionally halogenated C₁₋₄ alkoxy (e.g., methoxy, ethoxy, propoxy, butoxy, trifluoromethoxy, trifluoroethoxy and the like), formyl, C₂₋₄ alkanoyl (e.g., acetyl, propionyl and the like), C₁₋₄ alkyl sulfonyl (e.g., methane sulfonyl, ethane sulfonyl and the like) etc., and the number of such substituent groups is preferably 1 to 3.

The substituent group on the optionally substituted thiol group as a substituent group on R¹ includes groups exemplified above as the "substituent group on the optionally substituted hydroxyl group as a substituent group on R¹", among which preferable substituent groups include:
(1) optionally substituted alkyl (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower (C₁₋₆) alkyl);
(2) optionally substituted cycloalkyl (for example, C₃₋₇ cycloalkyl such as cyclopropyl, cyclobutyl, cyclopentyl, cyclohexyl and cycloheptyl);
(3) optionally substituted aralkyl (for example, phenyl-C₁₋₄ alkyl (e.g., benzyl, phenethyl etc.)); and
(4) optionally substituted aryl (for example, phenyl, naphthyl etc.).

The substituent groups which may be possessed by (1) optionally substituted alkyl, (2) optionally substituted cycloalkyl, (3) optionally substituted aralkyl and (4) optionally substituted aryl described above include halogens (e.g., fluorine, chlorine, bromine, iodine etc.), nitro, cyano, hydroxyl group, thiol group, amino group, carboxyl group, optionally halogenated C₁₋₄ alkyl (e.g., trifluoromethyl, methyl, ethyl etc.), optionally halogenated C₁₋₄ alkoxy (e.g., methoxy, ethoxy, propoxy, butoxy, trifluoromethoxy, trifluoroethoxy etc.), formyl, C₂₋₄ alkanoyl (e.g., acetyl, propionyl etc.) and C₁₋₄ alkyl sulfonyl (e.g., methane sulfonyl, ethane sulfonyl etc.), and the number of such substituent groups is preferably 1 to 3.

The substituent group on the optionally substituted amino group as a substituent group on R¹ is for example an amino group which may have 1 to 2 substituent groups exemplified as the "substituent group on the optionally substituted hydroxyl group as a substituent group on R¹", among which preferable substituent groups include:
(1) optionally substituted alkyl (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower (C₁₋₆) alkyl);
(2) optionally substituted cycloalkyl (for example, C₃₋₇ cycloalkyl such as cyclopropyl, cyclobutyl, cyclopentyl, cyclohexyl and cycloheptyl);
(3) optionally substituted alkenyl (for example, alkenyl containing 2 to 10 carbon atoms such as allyl, crotyl, 2-pentenyl and 3-hexenyl, preferably lower (C₂₋₆) alkenyl);
(4) optionally substituted cycloalkenyl (for example, cycloalkenyl containing 3 to 7 carbon atoms such as 2-cyclopentenyl, 2-cyclohexenyl, 2-cyclopentenyl methyl and 2-cyclohexenyl methyl);
(5) formyl or optionally substituted acyl (for example, alkanoyl containing 2 to 4 carbon atoms (e.g., acetyl, propionyl, butyryl, isobutyryl etc.) and C₁₋₄ alkyl sulfonyl (e.g., methane sulfonyl, ethane sulfonyl etc.); and
(6) optionally substituted aryl (for example, phenyl, naphthyl etc.).

The substituent groups which may be possessed by (1) optionally substituted alkyl, (2) optionally substituted cycloalkyl, (3) optionally substituted alkenyl, (4) optionally substituted cycloalkenyl, (5) optionally substituted acyl, and (6) optionally substituted aryl described above include halogens (e.g., fluorine, chlorine, bromine, iodine etc.), nitro, cyano, hydroxyl group, thiol group, amino group, carboxyl group, optionally halogenated C₁₋₄ alkyl (e.g., trifluoromethyl, methyl, ethyl etc.), optionally halogenated C₁₋₄ alkoxy (e.g., methoxy, ethoxy, propoxy, butoxy, trifluoromethoxy, trifluoroethoxy etc.), formyl, C₂₋₄ alkanoyl (e.g., acetyl, propionyl etc.) and C₁₋₄ alkyl sulfonyl (e.g., methane sulfonyl, ethane sulfonyl etc.), and the number of such substituent groups is preferably 1 to 3.

The substituent groups on the optionally substituted amino group as a substituent group on R¹ may be bound to each other to form a cyclic amino group, for example a cyclic amino group having a bond to the nitrogen atom thereof, which is formed by removing one hydrogen atom from the ring-constituting nitrogen atom in a 5- to 6-membered ring such as tetrahydropyrrole, piperazine, piperidine, morpholine, thiomorpholine, pyrrole or imidazole. The cyclic amino group may have substituent groups, and such substituent groups include halogens (e.g., fluorine, chlorine, bromine, iodine etc.), nitro, cyano, hydroxyl group, thiol group, amino group, carboxyl group, optionally halogenated C₁₋₄ alkyl (e.g., trifluoromethyl, methyl, ethyl etc.), optionally halogenated C₁₋₄ alkoxy (e.g., methoxy, ethoxy, propoxy, butoxy, trifluoromethoxy, trifluoroethoxy etc.), formyl, C₂₋₄ alkanoyl (e.g., acetyl, propionyl etc.) and C₁₋₄ alkyl sulfonyl (e.g., methane sulfonyl, ethane sulfonyl etc.), and the number of such substituent groups is preferably 1 to 3.

The optionally substituted acyl as a substituent group on R¹ includes those groups having a group selected from (1) to (6) below bound to a carbonyl group or sulfonyl group (e.g., acetyl, propionyl butyryl, isobutyryl, valeryl, isovaleryl, pivaloyl, hexanoyl, heptanoyl, octanonyl, cyclobutane carbonyl, cyclopentane carbonyl, cyclohexane carbonyl, cycloheptane carbonyl, crotonyl, 2-cyclohexene carbonyl, benzoyl, nicotinoyl, methane sulfonyl, ethane sulfonyl etc.):
(1) hydrogen;
(2) optionally substituted alkyl (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower (C₁₋₆) alkyl);
(3) optionally substituted cycloalkyl (for example, C₃₋₇ cycloalkyl such as cyclopropyl, cyclobutyl, cyclopentyl, cyclohexyl and cycloheptyl);
(4) optionally substituted alkenyl (for example, alkenyl containing 2 to 10 carbon atoms such as allyl, crotyl, 2-pentenyl and 3-hexenyl, preferably lower (C₂₋₆) alkenyl);
(5) optionally substituted cycloalkenyl (for example, cycloalkenyl containing 3 to 7 carbon atoms such as 2-cyclopentenyl, 2-cyclohexenyl, 2-cyclopentenyl methyl and 2-cyclohexenyl methyl); and
(6) optionally substituted 5- to 6-membered monocyclic aromatic group (for example, phenyl, pyridyl etc.), and the substituent groups which may be possessed by (2) optionally substituted alkyl, (3) optionally substituted cycloalkyl, (4) optionally substituted alkenyl, (5) optionally substituted cycloalkenyl, and (6) optionally substituted 5-to 6-membered monocyclic aromatic group described above include halogens (e.g., fluorine, chlorine, bromine, iodine etc.), nitro, cyano, hydroxyl group, thiol group, amino group, carboxyl group, optionally halogenated C₁₋₄ alkyl (e.g., trifluoromethyl, methyl, ethyl etc.), optionally halogenated C₁₋₄ alkoxy (e.g., methoxy, ethoxy, propoxy, butoxy, trifluoromethoxy, trifluoroethoxy etc.), formyl, C₂₋₄ alkanoyl (e.g., acetyl, propionyl etc.) and C₁₋₄ alkyl sulfonyl (e.g., methane sulfonyl, ethane sulfonyl etc.), and the number of such substituent groups is preferably 1 to 3.

The optionally esterified carboxyl group as a substituent group on R¹ includes those groups having a group selected from (1) to (6) below bound to a carbonyloxy group, preferably carboxyl, lower (C₁₋₆) alkoxycarbonyl, or aryloxycarbonyl (e.g., methoxycarbonyl, ethoxycarbonyl, propoxycarbonyl, phenoxycarbonyl, naphthoxycarbonyl etc.):
(1) hydrogen;
(2) optionally substituted alkyl (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower (C₁₋₆) alkyl);
(3) optionally substituted cycloalkyl (for example, C₃₋₇ cycloalkyl such as cyclopropyl, cyclobutyl, cyclopentyl, cyclohexyl and cycloheptyl);
(4) optionally substituted alkenyl (for example, alkenyl containing 2 to 10 carbon atoms such as allyl, crotyl, 2-pentenyl and 3-hexenyl, preferably lower (C₂₋₆) alkenyl);
(5) optionally substituted cycloalkenyl (for example, cycloalkenyl containing 3 to 7 carbon atoms such as 2-cyclopentenyl, 2-cyclohexenyl, 2-cyclopentenyl methyl and 2-cyclohexenyl methyl); and
(6) optionally substituted aryl (for example, phenyl, naphthyl etc.), and the substituent groups which may be possessed by (2) optionally substituted alkyl, (3) optionally substituted cycloalkyl, (4) optionally substituted alkenyl, (5) optionally substituted cycloalkenyl, and (6) optionally substituted aryl described above include halogens (e.g., fluorine, chlorine, bromine, iodine etc.), nitro, cyano, hydroxyl group, thiol group, amino group, carboxyl group, optionally halogenated C₁₋₄ alkyl (e.g., trifluoromethyl, methyl, ethyl etc.), optionally halogenated C₁₋₄ alkoxy (e.g., methoxy, ethoxy, propoxy, butoxy, trifluoromethoxy, trifluoroethoxy etc.), formyl, C₂₋₄ alkanoyl (e.g., acetyl, propionyl etc.) and C₁₋₄ alkyl sulfonyl (e.g., methane sulfonyl, ethane sulfonyl etc.), and the number of such substituent groups is preferably 1 to 3.

In the optionally substituted aromatic group as a substituent group on R¹, the aromatic group includes 5- to 6-membered, optionally heterocyclic aromatic groups such as phenyl, pyridyl, furyl, thienyl, pyrrolyl, imidazolyl, pyrazolyl, thiazolyl, oxazolyl, isothiazolyl, isoxazolyl, tetrazolyl, pyrazinyl, pyrimidinyl, pyridazinyl and triazolyl. The substituent groups on these aromatic groups include halogens (e.g., fluorine, chlorine, bromine, iodine etc.), nitro, cyano, hydroxyl group, thiol group, amino group, carboxyl group, optionally halogenated C₁₋₄ alkyl (e.g., trifluoromethyl, methyl, ethyl etc.), optionally halogenated C₁₋₄ alkoxy (e.g., methoxy, ethoxy, propoxy, butoxy, trifluoromethoxy, trifluoroethoxy etc.), formyl, C₂₋₄ alkanoyl (e.g., acetyl, propionyl etc.) and C₁₋₄ alkyl sulfonyl (e.g., methane sulfonyl, ethane sulfonyl etc.), and the number of such substituent groups is preferably 1 to 3.

The group R¹ may be substituted at any positions with the same or different kinds of 1 to 4 (preferably 1 to 2) substituent groups. When the "5- to 6-membered ring" in the "optionally substituted 5- to 6-membered ring" represented by R¹ has two or more substituent groups, two of the substituent groups may be bound to each other to form e.g. lower (C₁₋₆) alkylene (e.g., trimethylene, tetramethylene etc.), lower (C₁₋₆) alkyleneoxy (e.g., -CH₂-O-CH₂-, -O-CH₂-CH₂- etc.), lower (C₁₋₆) alkylene dioxy (e.g., -O-CH₂-O-, -O-CH₂-CH₂-O- etc.), lower (C₂₋₆) alkenylene (e.g., -CH₂-CH=CH-, -CH₂-CH₂-CH=CH-, -CH₂-CH=CH-CH₂- etc.) or lower (C₄₋₆) alkadienylene (e.g., -CH=CH-CH=CH- etc.).

In particular, the "substituent groups" which may be possessed by the "5- to 6-membered ring" in the "optionally substituted 5- to 6-membered ring" represented by R¹ include optionally halogenated or lower (C₁₋₄)-alkoxylated lower (C₁₋₄) alkyl (e.g., methyl, ethyl, t-butyl, trifluoromethyl, methoxymethyl, ethoxymethyl, propoxymethyl, butoxymethyl, methoxyethyl, ethoxyethyl, propoxyethyl, butoxyethyl etc.), optionally halogenated or lower (C₁₋₄)-alkoxylated lower (C₁₋₄) alkoxy (e.g., methoxy, ethoxy, propoxy, butoxy, t-butoxy, trifluoromethoxy, methoxymethoxy, ethoxymethoxy, propoxymethoxy, butoxymethoxy, methoxyethoxy, ethoxyethoxy, propoxyethoxy, butoxyethoxy, methoxypropoxy, ethoxypropoxy, propoxypropoxy, butoxypropoxy etc.), halogens (e.g., fluorine, chlorine etc.), nitro, cyano, amino which may be substituted with 1 to 2 lower (C₁₋₄) alkyl groups, formyl groups or lower (C₂₋₄) alkanoyl groups (e.g., amino, methyl amino, dimethyl amino, formyl amino, acetyl amino etc.), and 5- to 6-membered cyclic amino group (e.g., 1-pyrrolidinyl, 1-peperazinyl, 1-piperidinyl, 4-morpholino, 4-thiomorpholino, 1-imidazolyl, 4-tetrahydropyranyl etc.).

The "divalent group having 1 to 4 atoms in the linear-chain moiety thereof" represented by X¹ and X² includes, for example -(CH₂)ₐ,- [a' is an integer of 1 to 4 (preferably an integer of 1 to 2)], -(CH₂)_{b},-X³- [b' is an integer of 0 to 3 (preferably an integer of 0 to 1)] whereupon X³ represents an optionally substituted imino group (e.g., an imino group which may be substituted with lower (C₁₋₆) alkyl, lower (C₃₋₇) cycloalkyl, formyl, lower (C₂₋₇) alkanoyl or lower (C₁₋₆) alkoxy-carbonyl), a carbonyl group, an oxygen atom or an optionally oxidized sulfur atom (e.g., -S(O)ₘ- wherein m is an integer of 0 to 2)], -CH=CH-, -C≡C-, -CO-NH-, -SO₂-NH- etc. These groups may be bound to W via the right or left bond, but X¹ is bound to W preferably via the right bond, while X² is bound to W preferably via the left bond.

X¹ is preferably a bond, -(CH₂)_{b'}-O- [b' is an integer of 0, 1 or 2, preferably an integer of 0 to 1] or -C≡C-, more preferably a bond.

X² is preferably -(CH₂)ₐ,-[a' is an integer of 1 to 2], -(CH₂)_{b},-X³- [b' is an integer of 0 to 1, and X³ is an optionally substituted imino group, a carbonyl group, an oxygen atom or an optionally oxidized sulfur atom], -CH=CH-, -CO-NH- or -SO₂-NH-, more preferably -CO-NH-.

In the formula (I) above, the divalent groups W represented by the following formulae: 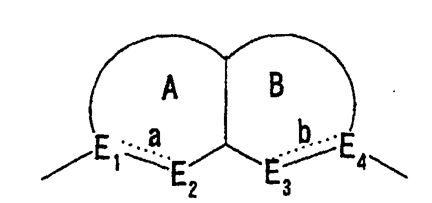 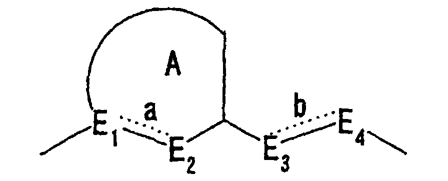 and 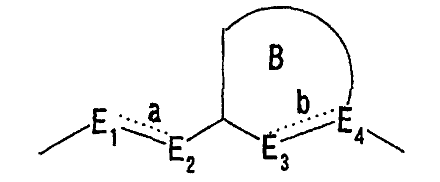 wherein rings A and B each represent an optionally substituted 5- to 7-membered ring, E₁ and E₄ each represent an optionally substituted carbon atom or an optionally substituted nitrogen atom, E₂ and E₃ each represent an optionally substituted carbon atom, an optionally substituted nitrogen atom, an optionally oxidized sulfur atom (e.g., -S(O)ₘ- (m is an integer of 0 to 2)) or an oxygen atom, and broken lines a and b each represent a single or double bond, are bound to their adjacent X¹ and X² in the following modes: 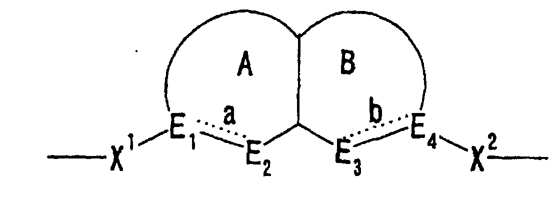 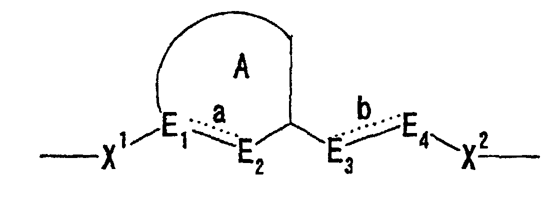 and 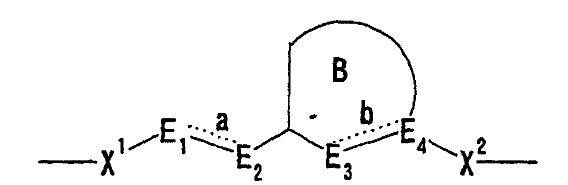 wherein each symbol has the same meaning as defined above.

In the "optionally substituted 5- to 7-membered ring" represented by A in the formula (I) above, the "5- to 7-membered ring" includes 5- to 7-membered (preferably 5- to 6-membered) saturated or unsaturated alicyclic hydrocarbons such as C₅₋₇ cycloalkane (e.g., cyclopentane, cyclohexane, cycloheptane etc.), C₅₋₇ cycloalkene (e.g., 1-cyclopentene, 2-cyclopentene, 3-cyclopentene, 2-cyclohexene, 3-cyclohexene etc.), C₅₋₆ cycloalkadiene (e.g., 2,4-cyclopentadiene, 2,4-cyclohexadiene, 2,5-cyclohexadiene etc.); 6-membered aromatic hydrocarbons such as benzene; 5-to 7-membered aromatic heterocyclic rings, saturated or unsaturated non-aromatic heterocyclic rings (aliphatic heterocyclic rings) containing one to three kinds (preferably one or two kinds) of at least one (preferably 1 to 4, more preferably 1 to 2) heteroatoms selected from an oxygen atom, a sulfur atom, a nitrogen atom etc.

As used herein, the "aromatic heterocyclic ring" includes 5- to 6-membered aromatic monocyclic heterocyclic rings (for example, furan, thiophene, pyrrole, oxazole, isoxazole, thiazole, isothiazole, imidazole, pyrazol, 1,2,3-oxadiazole, 1,2,4-oxadiazole, 1,3,4-oxadiazole, furazane, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine etc.), and the "non-aromatic heterocyclic ring" includes, for example, 5- to 7-membered (preferably 5- to 6-membered) saturated or unsaturated non-aromatic heterocyclic rings (aliphatic heterocyclic rigs) such as pyrrolidine, tetrahydrofuran, thiolan, piperidine, tetrahydropyran, morpholine, thiomorpholine, piperazine, pyran, oxepin, thiepin and azepin, or 5- to 6-membered non-aromatic heterocyclic rings comprising the above-described aromatic monocyclic heterocyclic rings wherein some or all double bonds have been saturated.

In the "optionally substituted 5- to 7-membered ring" represented by A, the "5- to 7-membered ring" is preferably a 5- to 6-membered aromatic ring, more preferably benzene, furan, thiophene, pyrrole or pyridine (preferably 6-membered ring), particularly preferably benzene.

The "substituent groups" which may be possessed by the "5- to 7-membered ring" in the "optionally substituted 5-to 7-membered ring" represented by A include those identical with the "substituent groups" which may be possessed by the "5- to 6-membered ring" in the "optionally substituted 5- to 6-membered ring" represented by R¹. The ring A may be substituted at any positions with the same or different kinds of 1 to 4 (preferably 1 to 2) substituent groups, that is, the ring may be substituted with substituent groups not only at the positions shown in E₁ and E₂ but also at any other replaceable positions.

In the "optionally substituted 5- to 7-membered ring" represented by B in the formula (I) above, the "5- to 7-membered ring" includes, for example, 5- to 7-membered rings which may have substituent groups at arbitrary replaceable positions, represented by the following chemical formula:

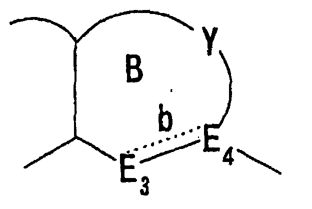

In the formula above, the divalent group represented by Y represents a divalent group forming a 5- to 7-membered, optionally substituted ring B, and examples thereof include:
(1) -(CH₂)ₐ₁-O-(CH₂)ₐ₂- (a1 and a2 are the same or different, and each represent 0, 1 or 2, provided that the sum of a1 and a2 is 2 or less), -O-(CH=CH)-,-(CH=CH)-O-;
(2) -(CH₂)_{b1}-S(O)ₘ-(CH₂)_{b2}- (m is an integer of 0 to 2, and b1 and b2 are the same or different, and each represent 0, 1 or 2, provided that the sum of b1 and b2 is 2 or less), - S(O)ₘ-(CH=CH)-, -(CH=CH)-S(O)ₘ-,
(3) -(CH₂)_{d1}- (d1 is 1, 2 or 3), -CH₂-(CH=CH)-, -(CH=CH)-CH₂-, -CH=CH-, -CH=,
(4) -(CH₂)ₑ₁-NH-(CH₂)ₑ₂- (e1 and e2 are the same or different, and each represent 0, 1 or 2, provided that the sum of e1 and e2 is 2 or less), -NH-(CH=CH)-, -(CH=CH)-NH-, -(CH₂)ₑ₆-(N=CH)-(CH₂)ₑ₇-, -(CH₂)ₑ₇-(CH=N)-(CH₂)ₑ₆- (one of e6 and e7 is 0, and the other is 0 or 1), -(CH₂)ₑ₈-(N=N)-(CH₂)ₑ₉- (one of e8 and e9 is 0, and the other is 0 or 1). Specific examples include divalent groups such as -O-, -O-CH₂-, -O-CH₂-CH₂-, -O-CH=CH-, -S(O)ₘ- (m is an integer of 0 to 2), - S(O)ₘ-CH₂- (m is an integer of 0 to 2), -S(O)ₘ-CH₂-CH₂- (m is an integer of 0 to 2), -S(O)ₘ-CH=CH- (m is an integer of 0 to 2), -CH₂-, -(CH₂)₂-, -(CH₂)₃-, -CH=, -CH=CH-, -CH=CH-CH₂-, -CH₂-CH=CH-, -NH-, -N=CH-, -CH=N-, and -N=N-, which are bonds originating from ring A.

Said divalent group may have substituent groups, and the substituent groups include oxo and groups exemplified above as the "substituent groups" which may be possessed by the "5- to 6-membered ring" in the "optionally substituted 5- to 6-membered cyclic ring" represented by R₁, among which lower (C₁₋₃) alkyl (e.g., methyl, ethyl, propyl etc.), phenyl, oxo and hydroxyl group are preferable. Further, the divalent group may be in any form such as -O-C(O)-which is a bond starting from ring A. Said divalent group may be substituted with the same or different kinds of 1 to 4 (preferably 1 to 2) substituent groups. The positions at which the divalent group is substituted may be any positions at which the substituent groups can be bound to the divalent group.

The divalent group represented by Y is preferably a group starting from ring A, such as -Y'-(CH₂)m'- (Y' is -S(O)ₘ- (m is an integer of 0 to 2), -O-, -NH- or -CH₂-, and m' is an integer of 0 to 2), -CH=, -CH=CH-, -N=CH-, -(CH₂)m'-Y'- (Y' is S(O)ₘ- (m is an integer of 0 to 2), -O-, -NH- or -CH₂-, and m' is an integer of 0 to 2), or -CH=N-, among which a group starting from ring A, such as -Y' -(CH₂)m'- (Y' is -S(O)ₘ- (m is an integer of 0 to 2), -O-, -NH- or -CH₂-, and m' is an integer of 0 to 2), -CH=, -CH=CH- and -N=CH- is preferable, and in particular a group starting from ring A, such as -Y'-(CH₂)₂- (Y' is -S(O)ₘ- (m is an integer of 0 to 2), -O-, -NH- or -CH₂-) (ring B is a 7-membered ring) is preferable.

The "substituent groups" which may be possessed by the "5- to 7-membered ring" in the "optionally substituted 5-to 7-membered ring" represented by B include oxo and groups exemplified above as the "substituent groups" which may be possessed by the "5- to 6-membered ring" in the "optionally substituted 5- to 6-membered ring" represented by R¹. The ring B may be substituted with the same or different kinds of 1 to 4 (preferably 1 to 2) substituent groups at any positions, but ring B is preferably unsubstituted at the E₃ position.

In preferable compounds of the formula (I) above, E₃ and E₄ are optionally substituted carbon atoms (preferably unsubstituted carbon atoms), and the broken line b is a double bond.

In the formula (Ia) above, the "optionally substituted 5- to 6-membered aromatic ring" represented by A' and the "optionally substituted 5- to 7-membered ring" represented by B' include rings exemplified as "the optionally substituted 5- to 6-membered aromatic ring" in the "optionally substituted 5- to 7-membered ring " represented by A and as the "optionally substituted 5- to 7-membered ring" represented by B, and the groups represented by W and W' are preferably the groups represented by the following formula: 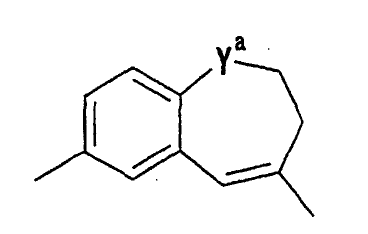 wherein Y^{a} represents an optionally substituted carbon atom, an optionally substituted nitrogen atom (e.g., -N(R^{a})-whereupon R^{a} represents a hydrogen atom, an optionally substituted hydrocarbon group or an optionally substituted acyl group), an optionally oxidized sulfur atom (e.g., -S(O)ₘ- whereupon m is an integer of 0 to 2), or an oxygen atom (preferably -CH₂-, -O-, -N(R^{a})- whereupon R^{a} represents a hydrogen atom, C₁₋₄ alkyl, formyl or C₂₋₅ alkanoyl) or -S(O)₂-.

The "divalent cyclic group" represented by Z¹ in the formula (I) above includes those groups formed by removing two hydrogen atoms from the groups exemplified as the "5-to 6-membered ring" in the "optionally substituted 5- to 6-membered cyclic group" represented by R¹, among which divalent cyclic groups formed by removing 2 hydrogen atoms from benzene, furan, thiophene, pyridine, cyclopentane, cyclohexane, pyrrolidine, piperidine, piperazine, morpholine, thiomorpholine and tetrahydropyran are preferable, and in particular divalent cyclic groups formed by removing 2 hydrogen atoms from benzene, cyclohexane and piperidine (preferably benzene) are more preferably used.

The "divalent cyclic group" represented by Z¹ may have the substituent groups exemplified as the "substituent group" which may be possessed by the "5- to 6-membered ring" in the "optionally substituted 5- to 6-membered cyclic group" represented by R¹, but preferably said divalent cyclic group does not have substituent groups other than those on X² and Z² and when Z¹ is a 6-membered divalent cyclic group (preferably phenylene), Z² is substituted preferably at a p-position relative to X².

In the formula (I) above, the "divalent group having 1 to 4 carbon atoms in the linear-chain moiety thereof" represented by Z² includes divalent groups having an optionally substituted hydrocarbon chain containing 1 to 4 carbon atoms (e.g., C₁₋₄ alkylene, C₂₋₄ alkenylene, preferably C₁₋₃ alkylene, more preferably methylene).

The divalent group represented by Z² may be any divalent chain whose linear-chain moiety contains 1 to 4 carbon atoms, for example an alkylene chain represented by -(CH₂)ₖ₁- (k1 is an integer of 1 to 4) or an alkenylene chain represented by -(CH₂)ₖ₂-(CH=CH)-(CH₂)ₖ₃- (k2 and k3 are the same or different and represent 0, 1 or 2 provided that the sum of k2 and k3 is 2 or less).

In the formula (Ia) above, the "divalent group having 1 to 4 carbon atoms in the linear-chain moiety thereof" represented by Z includes the groups exemplified as the "divalent group having 1 to 4 carbon atoms in the linear-chain moiety thereof" represented by Z² in the formula (I) above.

The divalent groups represented by X¹, X² and Z² may have substituent groups at arbitrary positions (preferably on carbon atoms), and such substituent groups may be any groups capable of binding to the divalent chain constituting the linear-chain moiety, and examples thereof include lower (C₁₋₆) alkyl (e.g., methyl, ethyl, propyl, isopropyl, butyl, isobutyl, sec-butyl, tert-butyl, pentyl, isopentyl, neopentyl and hexyl), lower (C₃₋₇) cycloalkyl (e.g., cyclopropyl, cyclobutyl, cyclopentyl, cyclohexyl and cycloheptyl), formyl, lower (C₂₋₇) alkanoyl (e.g., acetyl, propionyl and butyryl), an optionally esterified phosphono group, an optionally esterified carboxyl group, a hydroxyl group, oxo etc., among which lower alkyl containing 1 to 6 carbon atoms (preferably C₁₋₃ alkyl), hydroxyl group and oxo are particularly preferable.

The optionally esterified phosphono group includes those groups represented by -P(O)(OR⁷)(OR⁸) wherein R⁷ and R⁸ each represent hydrogen, a alkyl group containing 1 to 6 carbon atoms or a cycloalkyl group containing 3 to 7 carbon atoms, and R⁷ and R⁸ may be mutually bound to form a 5- to 7-membered ring.

In the formula above, the alkyl groups containing 1 to 6 carbon atoms represented by R⁷ and R⁸ include methyl, ethyl, propyl, isopropyl, butyl, isobutyl, sec-butyl, tert-butyl, pentyl, isopentyl, neopentyl and hexyl, the cycloalkyl groups containing 3 to 7 carbon atoms represented by R⁷ and R⁸ include cyclopropyl, cyclobutyl, cyclopentyl, cyclohexyl and cycloheptyl, preferably linear lower alkyl containing 1 to 6 carbon atoms, more preferably lower alkyl containing 1 to 3 carbon atoms. The groups represented by R⁷ and R⁸ may be the same or different, preferably the same. When R⁷ and R⁸ are mutually bound to form a 5- to 7-membered ring, R⁷ and R⁸ are mutually bound to form a linear C₂₋₄ alkylene side chain such as -(CH₂)₂-, -(CH₂)₃- or -(CH₂)₄-. The side chain may have substituent groups, and such substituent groups include a hydroxyl group, halogen etc.

As the optionally esterified carboxyl group, the esterified carboxyl group includes those carboxyl groups having a alkyl containing 1 to 6 carbon atoms or cycloalkyl group containing 3 to 7 carbon atoms bound thereto, for example, methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl etc.

The divalent group represented by Z² is preferably optionally substituted C₁₋₃ alkylene, particularly preferably C₁₋₃ alkylene optionally substituted with C₁₋₃ alkyl, hydroxyl group or oxo.

Further, the divalent group represented by Z² is a group starting from the benzene ring and represented by -Z'-(CH₂)ₙ- or -(CH₂)ₙ-Z'- (Z' represents -CH(OH)-, -C(O)- or -CH₂-, n is an integer of 0 to 2, and each of methylene groups may have the same or different kinds of 1 to 2 substituent groups), particularly preferably -Z'-(CH₂)ₙ- starting from the benzene ring (Z' represents -CH(OH)-, -C(O)- or -CH₂-, n is an integer of 0 to 2 (n is preferably 0), and each of methylene groups may have the same or different kinds of 1 to 2 substituent groups), most preferably methylene.

In the formula (I) above, the "amino group" in the "optionally substituted amino group whose nitrogen atom may have been converted into quaternary ammonium or oxide" represented by R² includes an amino group which may have 1 to 2 substituent groups or an amino group whose nitrogen atom has been converted into quaternary ammonium, which has 3 substituent groups. When there are two or more substituent groups on the nitrogen atom, the substituent groups may be the same or different, and when there are three substituent groups on the nitrogen atom, the amino group be in any forms such as -N⁺R³, -N⁺R²R' and -N⁺RR'R'' (R, R' and R'' are different and represent hydrogen or a substituent group). Further, the counter anion for the amino group whose nitrogen atom has been converted into quaternary ammonium includes halogen anions (e.g., Cl⁻, Br⁻, I⁻ etc.), anions derived from inorganic acids such as hydrochloric acid, hydrobromic acid, nitric acid, sulfuric acid and phosphoric acid, anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, propionic acid, stearic acid, sorbic acid, oleic acid, benzoic acid, lactic acid, glycolic acid, gluconic acid, dehydroacetic acid, erysorbic acid, taurocholic acid, nonanoic acid, hydroxynaphthoic acid, deoxycholic acid, cholic acid, capric acid, caproic acid, caprylic acid, laurylsulfuric acid, taurine, salicylic acid, myristic acid, palmitic acid, linolic acid, methasulfobenzoic acid, dioctylsulfosuccinic acid, tridecylsulfosuccinic acid, dibutylsulfosuccinic acid, dipalmitoyl phosphatidic acid, cinnamic acid, pentylsulfuric acid, heptylsulfuric acid, octylsulfuric acid, nonylsulfuric acid, 5-sulfoisophthalic acid, hexadecylsulfuric acid, tetradecylsulfuric acid, decylsulfuric acid, dodecylsulfuric acid, lauric acid, ethylsulfuric acid, laurylphosphoric acid and 3-sulfobenzoic acid (preferably salicylic acid, deoxycholic acid, myristic acid, dodecylsulfuric acid, decylsulfuric acid, tetradecylsulfuric acid, lauric acid etc.), and anions derived from acidic amino acids such as aspartic acid and glutamic acid, among which Cl⁻, Br⁻, I⁻ etc. are preferable. The anions may also be those derived from organic acids.

The substituent groups on said amino group include:
(1) optionally substituted alkyl (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower (C₁₋₆) alkyl);
(2) optionally substituted cycloalkyl (for example, C₃₋₈ cycloalkyl such as cyclopropyl, cyclobutyl, cyclopentyl, cyclohexyl, cycloheptyl and cycloctyl),
   wherein (2-1) said cycloalkyl contains one heteroatom selected from a sulfur atom, an oxygen atom and a nitrogen atom, and may form oxirane, thiolan, aziridine, tetrahydrofuran, tetrahydrothiophene, pyrrolidine, tetrahydrofuran, tetrahydrothiopyran, tetrahydrothiopyran 1-oxide, piperidine etc. (preferably 6-membered tetrahydropyran, tetrahydrothiopyran, piperidine etc.), and the position at which the cycloalkyl is bound to the amino group is preferably the 3- or 4-position (preferably the 4-position);
   (2-2) the cycloalkyl may also be condensed with a benzene ring to form indane (e.g., indan-1-yl, indan-2-yl), tetrahydronaphthalene (e.g., tetrahydronaphthalen-5-yl, tetrahydronaphthalen-6-yl etc.) (preferably indane); and
   (2-3) the cycloalkyl may also be crosslinked via a C₁₋₂ linear atomic chain to form a crosslinked cyclic hydrocarbon residue such as bicyclo[2.2.1] heptyl, bicyclo[2.2.2] octyl, bicylo[3.2.1] octyl and bicyclo[3.2.2] nonyl (preferably cyclohexyl having a crosslinkage via a linear atomic chain containing 1 to 2 carbon atoms, more preferably bicyclo[2.2.1] heptyl);
(3) optionally substituted alkenyl (for example, alkenyl containing 2 to 10 carbon atoms such as allyl, crotyl, 2-pentenyl and 3-hexenyl, preferably lower (C₂₋₆) alkenyl);
(4) optionally substituted cycloalkenyl (for example, C₃₋₇ cycloalkenyl containing 3 to 7 carbon atoms such as 2-cyclopentenyl, 2-cyclohexenyl, 2-cyclopentenyl methyl and 2-cyclohexenyl methyl);
(5) optionally substituted aralkyl (for example, phenyl-C₁₋₄ alkyl (e.g., benzyl, phenetyl etc.));
(6) formyl or optionally substituted acyl (for example, alkanoyl containing 2 to 4 carbon atoms (e.g., acetyl, propionyl, butyryl, isobutyryl etc.), alkyl sulfonyl containing 1 to 4 carbon atoms (e.g., methane sulfonyl, ethane sulfonyl etc.), alkoxy carbonyl containing 1 to 4 carbon atoms (e.g., methoxy carbonyl, ethoxy carbonyl, tert-butoxy carbonyl etc.), and aralkyloxy carbonyl containing 7 to 10 carbon atoms (e.g., benzyloxy carbonyl));
(7) optionally substituted aryl (for example, phenyl, naphthyl etc.);
(8) optionally substituted heterocyclic group (for example, groups formed by removing one hydrogen atom from 5- to 6-membered aromatic heterocyclic rings containing one or two kinds of 1 to 4 heteroatoms selected from a nitrogen atom, a sulfur atom and an oxygen atom, such as furan, thiophene, pyrrole, imidazole, pyrazol, thiazole, oxazole, isothiazole, isoxazole, tetrazole, pyridine, pyrazine, pyrimidine, pyridazine and triazole, or groups formed by removing one hydrogen atom from 5- to 6-membered non-aromatic heterocyclic ring containing one or two kinds of 1 to 4 heteroatoms selected from a nitrogen atom, a sulfur atom and an oxygen atom, such as tetrahydrofuran, tetrahydrothiophene, dithiolan, oxathiolan, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran and tetrahydropyran; preferably groups formed by removing one hydrogen atom from 5- to 6-membered non-aromatic heterocyclic rings; more preferably groups formed by removing one hydrogen atom from 5- to 6-membered non-aromatic heterocyclic rings containing one heteroatom, such as tetrahydrofuran, piperidine, tetrahydropyran and tetrahydrothiopyran. Further, the substituent groups on said amino group may be bound to each other to form 5- to 7-membered cyclic amino such as piperidine, piperazine, morpholine and thiomorpholine.

The substituent groups which may be possessed by (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl, (7) optionally substituted aryl, and (8) optionally substituted heterocyclic group described above include halogens (e.g., fluorine, chlorine, bromine, iodine etc.), optionally halogenated lower C₁₋₄ alkyl, optionally halogenated C₁₋₄ alkoxy (e.g., methoxy, ethoxy, propoxy, butoxy, trifluoromethoxy, trifluoroethoxy etc.), C₁₋₄ alkylene dioxy (e.g., -O-CH₂-O-, -O-CH₂-CH₂-O- etc.), formyl, C₂₋₄ alkanoyl (e.g., acetyl, propionyl etc.), C₁₋₄ alkyl sulfonyl (e.g., methane sulfonyl, ethane sulfonyl etc.), phenyl-lower (C₁₋₄) alkyl, C₃₋₇ cycloalkyl, cyano, nitro, hydroxyl group, thiol group, amino group, carboxyl group, lower (C₁₋₄) alkoxy-carbonyl, lower (C₇₋₁₀) aralkyloxy-carbonyl (preferably halogen, optionally halogenated lower (C₁₋₄) alkyl, optionally halogenated lower (C₁₋₄) alkoxy, phenyl-lower (C₁₋ ₄) alkyl, C₃₋₇ cycloalkyl, cyano, hydroxyl group etc.), and the number of such substituent groups is preferably 1 to 3.

In the formula (I) above, the "optionally substituted amino group whose nitrogen atom may have been converted into quaternary ammonium or oxide" represented by R² is preferably an amino group which may have 1 to 3 substituent groups selected from the following groups (1) to (5):
(1) linear or branched lower (C₁₋₆) alkyl which may have 1 to 3 substituent groups such as halogen, cyano, hydroxyl group or C₃₋₇ cycloalkyl;
(2) C₅₋₈ cycloalkyl (e.g., optionally substituted cyclopentyl, cyclohexyl, cycloheptyl, cycloctyl, tetrahydropyranyl, tetrahydrothiapyranyl, piperidinyl, indanyl, tetrahydronaphthalenyl, bicyclo[2.2.1] heptyl etc.) which may have 1 to 3 substituent groups such as halogens, optionally halogenated lower (C₁₋₄) alkyl or phenyl-lower lower (C₁₋₄) alkyl, may have one heteroatom selected from a sulfur atom, an oxygen atom and a nitrogen atom, may be condensed with a benzene ring, and may be crosslinked via a linear atomic chain containing 1 to 2 carbon atoms;
(3) phenyl-lower (C₁₋₄) alkyl which may have 1 to 3 substituent groups such as halogens, optionally halogenated lower (C₁₋₄) alkyl or optionally halogenated lower (C₁₋₄) alkoxy;
(4) phenyl which may have 1 to 3 substituent groups such as halogens, optionally halogenated lower (C₁₋₄) alkyl or optionally halogenated lower (C₁₋₄) alkoxy; and
(5) 5- to 6-membered aromatic heterocyclic groups (e.g., group formed by removing one hydrogen atom from furan, thiophene, pyrrole and pyridine) which may have 1 to 3 substituent groups such as halogens, optionally halogenated lower (C₁₋₄) alkyl, optionally halogenated lower (C₁₋₄) alkoxy, optionally halogenated lower (C₁₋₄) alkoxy-lower (C₁₋ ₄) alkoxy, phenyl-lower (C₁₋₄) alkyl, cyan or hydroxyl group.

In the formula (I) above, the "nitrogen-containing heterocyclic group" in the "optionally substituted nitrogen-containing heterocyclic group whose nitrogen atom may be converted into quaternary ammonium or oxide and which may contain a sulfur atom or oxygen atom as a ring-constituent atom" includes 5- to 6-membered aromatic heterocyclic rings which may contain one or two kinds of 1 to 3 heteroatoms selected from a nitrogen atom, a sulfur atom and an oxygen atom in addition to one nitrogen atom, such as pyrrole, imidazole, pyrazol, thiazole, oxazole, isothiazole, isoxazole, tetrazole, pyridine, pyrazine, pyrimidine, pyridazine and triazole, or 5- to 8-membered non-aromatic heterocyclic rings which may contain one or two kinds of 1 to 3 heteroatoms selected from a nitrogen atom, a sulfur atom and an oxygen atom in addition to one nitrogen atom, such as pyrrolidine, pyrroline, imidazolidine, imidazoline, pyrazolidine, pyrazoline, piperidine, piperazine, oxazine, oxadiazine, thiazine, thiadiazine, morpholine, thiomorpholine, azacycloheptane, azacycloctane (azocane) etc., and these nitrogen-containing heterocyclic rings may be crosslinked via a linear atomic chain containing 1 to 2 carbon atoms to form crosslinked nitrogen-containing heterocyclic rings such as azabicycio[2.2.1] heptane, azabicyclo[2.2.2] octane (quinuclidine) (preferably piperidine having a crosslinkage via a linear atomic chain containing 1 to 2 carbon atoms).

Preferable among the nitrogen-containing heterocyclic rings enumerated above are pyridine, imidazole, pyrrolidine, piperidine, piperazine, morpholine, thiomorpholine, azabicyclo[2.2.2] octane (preferably 6-membered rings).

The nitrogen atom in the "nitrogen-containing heterocyclic ring" may have been converted into quaternary ammonium or may have been oxidized. When the nitrogen atom in the "nitrogen-containing heterocyclic ring" has been converted into quaternary ammonium, the counter anion for the "nitrogen-containing heterocyclic group whose nitrogen atom has been converted into quaternary ammonium" includes halogen anions (e.g., Cl⁻, Br⁻, I⁻ etc.), anions derived from inorganic acids such as hydrochloric acid, hydrobromic acid, nitric acid, sulfuric acid and phosphoric acid, anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, propionic acid, stearic acid, sorbic acid, oleic acid, benzoic acid, lactic acid, glycolic acid, gluconic acid, dehydroacetic acid, erysorbic acid, taurocholic acid, nonanoic acid, hydroxynaphthoic acid, deoxycholic acid, cholic acid, capric acid, caproic acid, caprylic acid, laurylsulfuric acid, taurine, salicylic acid, myristic acid, palmitic acid, linolic acid, methasulfobenzoic acid, dioctylsulfosuccinic acid, tridecylsulfosuccinic acid, dibutylsulfosuccinic acid, dipalmitoyl phosphatidic acid, cinnamic acid, pentylsulfuric acid, heptylsulfuric acid, octylsulfuric acid, nonylsulfuric acid, 5-sulfoisophthalic acid, hexadecylsulfuric acid, tetradecylsulfuric acid, decylsulfuric acid, dodecylsulfuric acid, tetradecylsulfuric acid, lauric acid, ethylsulfuric acid, laurylphosphoric acid and 3-sulfobenzoic acid (preferably salicylic acid, deoxycholic acid, myristic acid, dodecylsulfuric acid, decylsulfuric acid, tetradecylsulfuric acid, lauric acid etc.), and anions derived from acidic amino acids such as aspartic acid and glutamic acid, among which Cl⁻, Br⁻, I⁻ etc. are preferable. The anions may also be those derived from organic acids.

The "nitrogen-containing heterocyclic group" may be bound via a carbon atom or nitrogen atom to the divalent group represented by Z². The "nitrogen-containing heterocyclic group" may be bound to the idvalent group represented by Z² at a ring-forming carbon atom such as in 2-pyridine, 3-pyridyl and 2-piperidinyl, preferably at a ring-forming nitrogen atom as shown in:

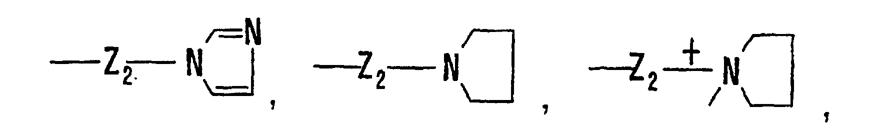

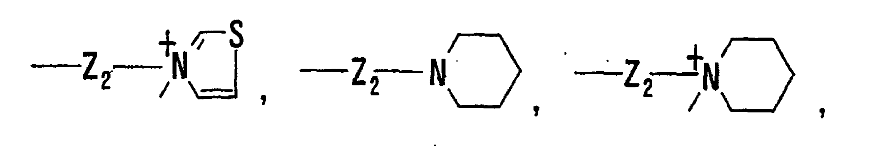

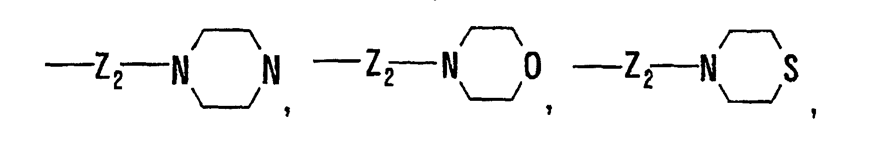

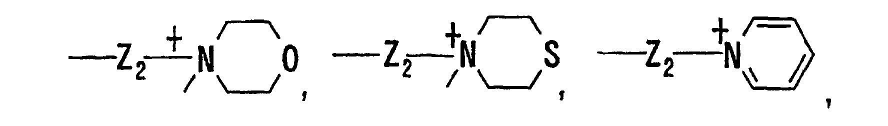

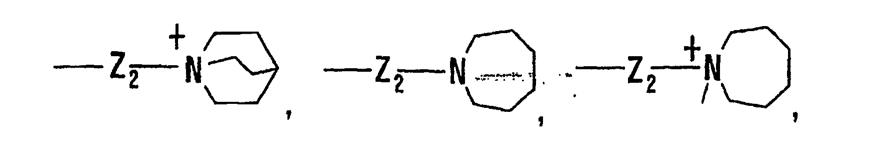

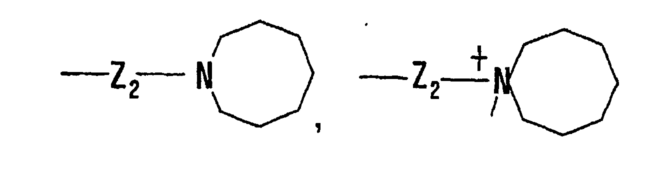

The substituent group which may be possessed by the "nitrogen-containing heterocyclic group" includes halogens (e.g., fluorine, chlorine, bromine, iodine etc.), optionally substituted lower (C₁₋₄) alkyl, optionally substituted lower (C₁₋₄) alkoxy, optionally substituted phenyl, optionally substituted mono- or diphenyl-lower (C₁₋ ₄) alkyl, optionally substituted C₃₋₇ cycloalkyl, cyano, nitro, hydroxyl group, thiol group, amino group, carboxyl group, lower (C₁₋₄) alkoxy-carbonyl, formyl, lower (C₂₋₄) alkanoyl, lower (C₁₋₄) alkyl sulfonyl, optionally substituted heterocyclic group (for example, groups formed by removing one hydrogen atom from 5- to 6-membered aromatic heterocyclic rings containing one or two kinds of 1 to 4 heteroatoms selected from a nitrogen atom, a sulfur atom and an oxygen atom, such as furan, thiophene, pyrrole, imidazole, pyrazol, thiazole, oxazole, isothiazole, isoxazole, tetrazole, pyridine, pyrazine, pyrimidine, pyridazine and triazole, or 5- to 6-membered non-aromatic heterocyclic rings containing one or two kinds of 1 to 4 heteroatoms selected from a nitrogen atom, a sulfur atom and an oxygen atom, such as tetrahydrofuran, tetrahydrothiophene, dithiolan, oxathiolan, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran and tetrahydrothiopyran, and the number of such substituent groups is preferably 1 to 3.

The substituent groups possessed by the "optionally substituted lower (C₁₋₄) alkyl", "optionally substituted lower (C₁₋₄) alkoxy", "optionally substituted phenyl", "optionally substituted mono- or diphenyl lower (C₁₋₄) alkyl", "optionally substituted C₃₋₇ cycloalkyl", and "optionally substituted heterocyclic group" as substituent groups which may be possessed by the "nitrogen-containing heterocyclic group" include, for example, halogens (e.g., fluorine, chlorine, bromine, iodine etc.), optionally halogenated lower (C₁₋₄) alkyl, lower (C₃₋₁₀) cycloalkyl, lower (C₃₋₁₀) cycloalkenyl, optionally halogenated C₁₋₄ alkoxy (e.g., methoxy, ethoxy, trifluoromethoxy, trifluoroethoxy etc.), formyl, C₂₋₄ alkanoyl (e.g., acetyl, propionyl etc.), C₁₋₄ alkyl sulfonyl (e.g., methane sulfonyl, ethane sulfonyl etc.), C₁₋₃ alkylene dioxy (e.g., methylene dioxy, ethylene dioxy etc.), cyano, nitro, hydroxyl group, thiol group, amino group, carboxyl group, and lower (C₁₋₄) alkoxy-carbonyl, and the number of such substituent groups is preferably 1 to 3.

In the formula (I), the substituent groups which may be possessed by the "nitrogen-containing heterocyclic group" in the "optionally substituted nitrogen-containing heterocyclic group whose nitrogen atom may be converted into quaternary ammonium or oxide and which may contain a sulfur atom or oxygen atom as a ring-constituent atom" include (1) halogen, (2) cyano, (3) hydroxyl group, (4) carboxyl group, (5) lower (C₁₋₄) alkoxy-carbonyl, (6) lower (C₁₋₄) alkyl which may be substituted with halogen, hydroxyl group or lower (C₁₋₄) alkoxy, (7) lower (C₁₋₄) alkoxy which may be substituted with halogen, hydroxyl group or lower (C₁₋₄) alkoxy, (8) phenyl which may be substituted with halogen, lower (C₁₋₄) alkyl, hydroxyl group, lower (C₁₋₄) alkoxy or C₁₋₃ alkylene dioxy, (9) mono- or diphenyl-lower (C₁₋₄) alkyl which may be substituted with halogen, lower (C₁₋₄) alkyl, hydroxyl group, lower (C₁₋₄) alkoxy or C₁₋₃ alkylene dioxy, or (10) groups formed by removing one hydrogen atom from 5- to 6-membered aromatic heterocyclic rings such as furan, thiophene, pyrrole and pyridine.

In the formula (I) above, the "group bound via a sulfur atom" represented by R² includes those groups represented by the formula -S(O)ₘ-R^{s} wherein m is an integer of 0 to 2, and R^{s} represents a substituent group. In the formula above, the substituent group represented by R^{s} includes for example:
(1) optionally substituted alkyl (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower (C₁₋₆) alkyl);
(2) optionally substituted cycloalkyl (for example, C₃₋₇ cycloalkyl such as cyclopropyl, cyclobutyl, cyclopentyl, cyclohexyl and cycloheptyl);
(3) optionally substituted aralkyl (for example, phenyl-C₁₋₄ alkyl (e.g., benzyl, phenetyl etc.));
(4) optionally substituted aryl (for example, phenyl, naphthyl etc.), and the substituent groups which may be possessed by (1) optionally substituted alkyl, (2) optionally substituted cycloalkyl, (3) optionally substituted aralkyl, and (4) optionally substituted aryl described above include halogens (e.g., fluorine, chlorine, bromine, iodine etc.), nitro, cyano, hydroxyl group, thiol group, amino group, carboxyl group, optionally halogenated C₁₋₄ alkyl (e.g., trifluoromethyl, methyl, ethyl etc.), optionally halogenated C₁₋₄ alkoxy (e.g., methoxy, ethoxy, trifluoromethoxy, trifluoroethoxy etc.), formyl, C₂₋₄ alkanoyl (e.g., acetyl, propionyl etc.), C₁₋₄ alkyl sulfonyl (e.g., methane sulfonyl, ethane sulfonyl etc.) etc., and the number of such substituent groups is preferably 1 to 3.

In the group represented by R² in the formula (I) above, that is, in the "group represented by the formula: 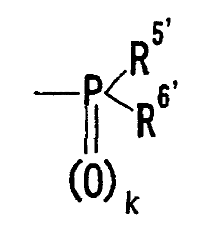 wherein k is 0 or 1, and when k is 0, the phosphorus atom may form a phosphonium salt, R⁵' and R⁶' each represent an optionally substituted hydrocarbon group, an optionally substituted hydroxy group or an optionally substituted amino group, and R⁵' and R⁶' may be bound to each other to form a cyclic group with their adjacent phosphorus atom", the "hydrocarbon group" in the optionally substituted hydrocarbon group represented by R⁵' and R^{6'} includes:
(1) optionally substituted alkyl (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower (C₁₋₆) alkyl);
(2) optionally substituted cycloalkyl (for example, C₃₋₇ cycloalkyl such as cyclopropyl, cyclobutyl, cyclopentyl, cyclohexyl and cycloheptyl);
(3) optionally substituted alkenyl (for example, alkenyl containing 2 to 10 carbon atoms such as allyl, crotyl, 2-pentenyl and 3-hexenyl, preferably lower (C₂₋₆) alkenyl);
(4) optionally substituted cycloalkenyl (for example, cycloalkenyl containing 3 to 7 carbon atoms such as 2-cyclopentenyl, 2-cyclohexenyl, 2-cyclopentenyl methyl and 2-cyclohexenyl methyl);
(5) optionally substituted alkynyl (for example, alkynyl containing 2 to 10 carbon atoms such as ethynyl, 1-propynyl, 2-propynyl, 1-butynyl, 2-pentynyl and 3-hexynyl, preferably lower (C₂₋₆) alkynyl);
(6) optionally substituted aralkyl (for example, phenyl-C₁₋₄ alkyl (e.g., benzyl, phenetyl etc.)); and
(7) optionally substituted aryl (for example, phenyl, naphthyl etc.), and the substituent groups which may be possessed by (1) optionally substituted alkyl, (2) optionally substituted cycloalkyl, (3) optionally substituted alkenyl, (4) optionally substituted cycloalkenyl, (5) optionally substituted alkynyl, (6) optionally substituted aralkyl and (7) optionally substituted aryl described above include halogens (e.g., fluorine, chlorine, bromine, iodine etc.), nitro, cyano, hydroxyl group, thiol group, amino group, carboxyl group, optionally halogenated C₁₋₄ alkyl (e.g., trifluoromethyl, methyl, ethyl etc.), optionally halogenated C₁₋₄ alkoxy (e.g., methoxy, ethoxy, trifluoromethoxy, trifluoroethoxy etc.), formyl, C₂₋₄ alkanoyl (e.g., acetyl, propionyl etc.) and C₁₋₄ alkyl sulfonyl (e.g., methane sulfonyl, ethane sulfonyl etc.), and the number of such substituent groups is preferably 1 to 3.

The "optionally substituted hydroxyl group" represented by R⁵' and R⁶' includes hydroxyl groups which may possess:
(1) optionally substituted alkyl (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower (C₁₋₆) alkyl) ;
(2) optionally substituted cycloalkyl (for example, C₃₋₇ cycloalkyl such as cyclopropyl, cyclobutyl, cyclopentyl, cyclohexyl and cycloheptyl);
(3) optionally substituted alkenyl (for example, alkenyl containing 2 to 10 carbon atoms such as allyl, crotyl, 2-pentenyl and 3-hexenyl, preferably lower (C₂₋₆) alkenyl);
(4) optionally substituted cycloalkenyl (for example, cycloalkenyl containing 3 to 7 carbon atoms such as 2-cyclopentenyl, 2-cyclohexenyl, 2-cyclopentenyl methyl and 2-cyclohexenyl methyl);
(5) optionally substituted aralkyl (for example, phenyl-C₁₋₄ alkyl (e.g., benzyl, phenetyl etc.));
(6) formyl or optionally substituted acyl (for example, alkanoyl containing 2 to 4 carbon atoms (e.g., acetyl, propionyl, butyryl, isobutyryl etc.), alkyl sulfonyl containing 1 to 4 carbon atoms (e.g., methane sulfonyl, ethane sulfonyl etc.); and
(7) optionally substituted aryl (for example, phenyl, naphthyl etc.).

The substituent groups which may be possessed by (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl, and (7) optionally substituted aryl described above include halogens (e.g., fluorine, chlorine, bromine, iodine etc.), nitro, cyano, hydroxyl group, thiol group, amino group, carboxyl group, optionally halogenated C₁₋₄ alkyl (e.g., trifluoromethyl, methyl, ethyl etc.), optionally halogenated C₁₋₄ alkoxy (e.g., methoxy, ethoxy, trifluoromethoxy, trifluoroethoxy etc.), formyl, C₂₋₄ alkanoyl (e.g., acetyl, propionyl etc.) and C₁₋₄ alkyl sulfonyl (e.g., methane sulfonyl, ethane sulfonyl etc.), and the number of such substituent groups is preferably 1 to 3.

In the formula above, R⁵' and R⁶' may be mutually bound to form a cyclic group (preferably a 5- to 7-membered ring) together with their adjacent phosphorus atom. The cyclic group may have substituent groups, and such substituent groups include halogens (e.g., fluorine, chlorine, bromine, iodine etc.), nitro, cyano, hydroxyl group, thiol group, amino group, carboxyl group, optionally halogenated C₁₋₄ alkyl (e.g., trifluoromethyl, methyl, ethyl etc.), optionally halogenated C₁₋₄ alkoxy (e.g., methoxy, ethoxy, trifluoromethoxy, trifluoroethoxy etc.), formyl, C₂₋₄ alkanoyl (e.g., acetyl, propionyl etc.) and C₁₋₄ alkyl sulfonyl (e.g., methane sulfonyl, ethane sulfonyl etc.), and the number of such substituent groups is preferably 1 to 3.

In the formula (I) above, the counter anion for the phosphorus atom to form a phosphonium salt includes halogen anions (e.g., Cl⁻, Br⁻, I⁻ etc.), anions derived from inorganic acids such as hydrochloric acid, hydrobromic acid, nitric acid, sulfuric acid and phosphoric acid, anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, propionic acid, stearic acid, sorbic acid, oleic acid, benzoic acid, lactic acid, glycolic acid, gluconic acid, dehydroacetic acid, erysorbic acid, taurocholic acid, nonanoic acid, hydroxynaphthoic acid, deoxycholic acid, cholic acid, capric acid, caproic acid, caprylic acid, laurylsulfuric acid, taurine, salicylic acid, myristic acid, palmitic acid, linolic acid, methasulfobenzoic acid, dioctylsulfosuccinic acid, tridecylsulfosuccinic acid, dibutylsulfosuccinic acid, dipalmitoyl phosphatidic acid, cinnamic acid, pentylsulfuric acid, heptylsulfuric acid, octylsulfuric acid, nonylsulfuric acid, 5-sulfoisophthalic acid, hexadecylsulfuric acid, tetradecylsulfuric acid, decylsulfuric acid, dodecylsulfuric acid, tetradecylsulfuric acid, lauric acid ethylsulfuric acid, laurylphosphoric acid and 3-sulfobenzoic acid (preferably salicylic acid, deoxycholic acid, myristic acid, dodecylsulfuric acid, decylsulfuric acid, tetradecylsulfuric acid, lauric acid etc.), and anions derived from acidic amino acids such as aspartic acid and glutamic acid, among which Cl⁻, Br⁻, I⁻ etc. are preferable. The anions may also be those derived from organic acids.

The optionally substituted amino group represented by R⁵' and R⁶' includes amino groups which may have 1 or 2 groups selected from:
(1) optionally substituted alkyl (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower (C₁₋₆) alkyl);
(2) optionally substituted cycloalkyl (for example, C₃₋₇ cycloalkyl such as cyclopropyl, cyclobutyl, cyclopentyl, cyclohexyl and cycloheptyl);
(3) optionally substituted alkenyl (for example, alkenyl containing 2 to 10 carbon atoms such as allyl, crotyl, 2-pentenyl and 3-hexenyl, preferably lower (C₂₋₆) alkenyl);
(4) optionally substituted cycloalkenyl (for example, cycloalkenyl containing 3 to 7 carbon atoms such as 2-cyclopentenyl, 2-cyclohexenyl, 2-cyclopentenyl methyl and 2-cyclohexenyl methyl);
(5) formyl or optionally substituted acyl (for example, alkanoyl containing 2 to 4 carbon atoms (e.g., acetyl, propionyl, butyryl, isobutyryl etc.), alkyl sulfonyl containing 1 to 4 carbon atoms (e.g., methane sulfonyl, ethane sulfonyl etc.)); and
(6) optionally substituted aryl (for example, phenyl, naphthyl etc.).

The substituent groups which may be possessed by (1) optionally substituted alkyl, (2) optionally substituted cycloalkyl, (3) optionally substituted alkenyl, (4) optionally substituted cycloalkenyl, (5) optionally substituted acyl, and (6) optionally substituted aryl described above include halogens (e.g., fluorine, chlorine, bromine, iodine etc.), nitro, cyano, hydroxyl group, thiol group, amino group, carboxyl group, optionally halogenated C₁₋₄ alkyl (e.g., trifluoromethyl, methyl, ethyl etc.), optionally halogenated C₁₋₄ alkoxy (e.g., methoxy, ethoxy, trifluoromethoxy, trifluoroethoxy etc.), formyl, C₂₋₄ alkanoyl (e.g., acetyl, propionyl etc.) and C₁₋₄ alkyl sulfonyl (e.g., methane sulfonyl, ethane sulfonyl etc.), and the number of such substituent groups is preferably 1 to 3.

The substituent groups in the "optionally substituted amidino group" and "optionally substituted guanidino group" represented by R² include those exemplified above as the substituent groups on the "optionally substituted amino group whose nitrogen atom may be converted into quaternary ammonium or oxide" represented by R².

R² is preferably (1) optionally substituted amino group whose nitrogen atom may be converted into quaternary ammonium or oxide, (2) optionally substituted nitrogen-containing heterocyclic group whose nitrogen atom may be converted into quaternary ammonium or oxide and which may contain a sulfur atom or oxygen atom as a ring constituent atom, (3) optionally substituted amidino group or (4) optionally substituted guanidino group, and R² is more preferably an optionally substituted amino group whose nitrogen atom may be converted into quaternary ammonium. Further, R² may be an optionally substituted amidino group or an optionally substituted guanidino group.

R² is more preferably a group represented by the formula -NRR'' or -N⁺RR'R'' wherein R, R' and R'' each represent an optionally substituted aliphatic hydrocarbon group or an optionally substituted alicyclic heterocyclic group.

In the formula above, the "optionally substituted aliphatic hydrocarbon group (non-aromatic hydrocarbon group)" and "optionally substituted alicyclic heterocyclic group (non-aromatic heterocyclic group)" represented by R, R' and R'' include groups identical with the "optionally substituted aliphatic hydrocarbon group" and "optionally substituted alicyclic heterocyclic group" exemplified as the substituent group which may be possessed by the "optionally substituted amino group" represented by the substituent group R².

In particular, R and R' are preferably optionally substituted linear hydrocarbon groups, more preferably optionally substituted C₁₋₆ alkyl groups, particularly preferably optionally substituted methyl groups.

R" is preferably an optionally substituted alicyclic hydrocarbon group (preferably an optionally substituted C₃₋₈ cycloalkyl group, more preferably an optionally substituted cyclohexyl) or an optionally substituted alicyclic heterocyclic group (preferably an optically substituted, saturated alicyclic heterocyclic group (preferably 6-membered ring), more preferably optionally substituted tetrahydropyranyl, optionally substituted tetrahydrothiopyranyl or optionally substituted piperidyl, particularly preferably optionally substituted tetrahydropyranyl).

The compounds represented by formula (I) are preferably the compounds shown below:
N,N-dimethyl-N-(4-(((2-(4-methylphenyl)-6,7-dihydro-5H-benzocyclohepten-8-yl)carbonyl)amino)benzyl)-N-(4-tetrahydropyranyl)ammonium chloride;
N-methyl-N-[4-[[[2-(4-methylphenyl)-6,7-dihydro-5H-benzocyclohepten-8-yl] carbonyl] amino] benzyl] piperidinium iodide;
N-methyl-N-[4-[[[7-(4-methylphenyl)-2,3-dihydro-1-benzoxepin-4-yl]carbonyl]amino]benzyl] piperidinium iodide; N-[4-[N-methyl-N-(tetrahydropyran-4-yl)aminomethyl]phenyl]-7-(4-methylphenyl)-2,3-dihydro-1-benzoxepin-4-carboxamide; N-[4-[N-methyl-N-(tetrahydropyran-4-yl)aminomethyl]phenyl]-7-(4-morpholinophenyl)-2,3-dihydro-1-benzoxepin-4-carboxamide;
7-(4-ethoxyphenyl)-N-[4-[N-methyl-N-(tetrahydropyran-4-yl) aminomethyl]phenyl]-2,3-dihydro-1-benzoxepin-4-carboxamide; N,N-dimethyl-N-[4-[[[2-(4-methylphenyl)-6,7-dihydro-5H-benzocyclohepten-8-yl]carbonyl]amino]benzyl]-N-(tetrahydropyran-4-yl)ammonium iodide; N-methyl-N-[4-[[[7-(4-methylphenyl)-3,4-dihydronaphthalen-2-yl]carbonyl]amino]benzyl]piperidinium iodide;
N,N-dimethyl-N-(((7-(4-methylphenyl)-2,3-dihydro-1-benzoxepin-4-yl)carbonyl)amino)benzyl)-N-(4-oxocyclohexyl)ammonium chloride; N-(4-(((7-(4-ethoxyphenyl)-2,3-dihydro-1-benzoxepin-4-yl) carbonyl)amino)benzyl)-N,N-dimethyl-N-(4-tetrahydropyranyl)ammonium chloride;
N-[4-[N-methyl-N-(tetrahydropyran-4-yl)aminomethyl] phenyl]-7-(4-propoxyphenyl)-1,1-dioxo-2,3-dihydro-1-benzothiepin-4-carboxamide;
7-(4-butoxyphenyl)-N-[4-[N-methyl-N-(tetrahydropyran-4-yl) aminomethyl]phenyl]-1,1-dioxo-2,3-dihydro-1-benzothiepin-4-carboxamide;
7-[4-[N-methyl-N-(2-propoxyethyl)amino]phenyl]-N-[4-[[N-methyl-N-(tetrahydropyran-4-yl)amino]methyl]phenyl]-1,1-dioxo-2,3-dihydro-1-benzothiepin-4-carboxamide;
7-[4-(2-ethoxyethoxy)phenyl)-N-[4-[[N-methyl-N-(tetrahydropyran-4-yl)amino]methyl]phenyl]-1,1-dioxo-2,3-dihydro-1-benzothiepin-4-carboxamide;
N-[4-[[N-methyl-N-(tetrahydropyran-4-yl)amino]methyl] phenyl] -7- [4- (2-propoxyethoxy) phenyl]-1,1-dioxo-2, 3-dihydro-1-benzothiepin-4-carboxamide; 7-[4-(2-butoxyethoxy)phenyl)-N-[4-[[N-methyl-N-(tetrahydropyran-4-yl)amino]methyl]phenyl]-1,1-dioxo-2,3-dihydro-1-benzothiepin-4-carboxamide;
7-[4-(2-ethoxyethoxy)-3,5-dimethylphenyl]-N-[4-[[N-methyl-N-(tetrahydro-2H-pyran-4-yl)amino]methyl]phenyl]-1,1-dioxo-2,3-dihydro-1-benzothiepin-4-carboxamide;
7-(4-ethoxyethoxyphenyl)-1-ethyl-N-[4-[[N-methyl-N-(tetrahydropyran-4-yl)amino]methyl]phenyl]-2,3-dihydro-1-benzoazepin-4-carboxylic acid amide;
1-ethyl-7-(4-propoxyethoxyphenyl)-N-[4-[[N-methyl-N-(tetrahydropyran-4-yl)amino]methyl]phenyl]-2,3-dihydro-1-benzoazepin-4-carboxylic acid amide; 7-(4-butoxyethoxyphenyl)-1-ethyl-N-[4-[[N-methyl-N-(tetrahydropyran-4-yl]amino]methyl]phenyl]-2,3-dihydro-1-benzoazepin-4-carboxylic acid amide;
7-(4-ethoxyethoxyphenyl)-1-formyl-N-[4-[[N-methyl-N-(tetrahydropyran-4-yl)amino]methyl]phenyl]-2,3-dihydro-1-benzoazepin-4-carboxylic acid amide;
1-formyl-7-(4-propoxyethoxyphenyl)-N-[4-[[N-methyl-N-(tetrahydropyran-4-yl)amino]methyl]phenyl]-2,3-dihydro-1-benzoazepin-4-carboxylic acid amide;
7-(4-butoxyethoxyphenyl)-1-formyl-N-[4-[[N-methyl-N-(tetrahydropyran-4-yl)amino]methyl]phenyl]-2,3-dihydro-1-benzoazepin-4-carboxylic acid amide;
7-[4-(2-butoxyethoxy)phenyl)-N-[4-[[N-methyl-N-(tetrahydropyran-4-yl)amino]methyl]phenyl]-1-propyl-2,3-dihydro-1-benzoazepin-4-carboxylic acid amide;
N-[4-[N-methyl-N-(tetrahydropyran-4-yl)aminomethyl] phenyl]-7-[2-(4-propoxyphenyl)ethoxy]-1,1-dioxo-2,3-dihydro-1-benzothiepin-4-carboxamide;
N-[4-[N-methyl-N-(tetrahydropyran-4-yl)aminomethyl] phenyl]-7-[(3-propoxybenzyl)oxy]-1,1-dioxo-2,3-dihydro-1-benzothiepin-4-carboxamide;
N-[4-[N-methyl-N-(tetrahydropyran-4-yl)aminomethyl] phenyl]-7-[(2-propoxybenzyl)oxy]-1,1-dioxo-2,3-dihydro-1-benzothiepin-4-carboxamide;
N-[4-[N-methyl-N-(tetrahydropyran-4-yl)aminomethyl] phenyl]-7-[(2-chlorobenzyl)oxy]-1,1-dioxo-2,3-dihydro-1-benzothiepin-4-carboxamide;
N-[4-[N-methyl-N-(tetrahydropyran-4-yl) aminomethyl] phenyl]-7-[(4-ethoxybenzyl)oxy]-1,1-dioxo-2,3-dihydro-1-benzothiepin-4-carboxamide;
N-[4-[N-methyl-N-(tetrahydropyran-4-yl) aminomethyl] phenyl]-7-[[4-(propoxymethyl)benzyl]oxy]-1,1-dioxo-2,3-dihydro-1-benzothiepin-4-carboxamide; N-[1-(tetrahydropyran-4-yl)piperidin-4-yl]-7-(4-methylphenyl)-2,3-dihydro-1-benzoxepin-4-carboxamide; and
N-[4-[(2-imidazolin-2-yl)methyl]phenyl]-7-(4-methylphenyl)-2,3-dihydro-1-benzoxepin-4-carboxamide.

The salt of the "compound having CCR5 antagonism" used in this invention is preferably a pharmacologically acceptable salt, for example a salt with an inorganic base, a salt with an organic base, a salt with an inorganic acid, a salt with an organic acid, or a salt with a basic or acidic amino acid. Preferable examples of the salt with an inorganic base include alkali metal salts such as sodium salts, potassium salts etc.; alkaline earth metal salts such as calcium salts, magnesium salts etc.; and aluminum salts, ammonium salts etc. Preferable examples of the salt with an organic base include salts with trimethylamine, triethylamine, pyridine, picoline, ethanolamine, diethanolamine, triethanolamine, dicyclohexylamine, N,N'-dibenzylethylenediamine etc. Preferable examples of the salt with an inorganic acid include salts with hydrochloric acid, hydrobromic acid, nitric acid, sulfuric acid and phosphoric acid. Preferable examples of the salt with an organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, propionic acid, stearic acid, sorbic acid, oleic acid, benzoic acid, lactic acid, glycolic acid, gluconic acid, dehydroacetic acid, erysorbic acid, taurocholic acid, nonanoic acid, hydroxynaphthoic acid, deoxycholic acid, cholic acid, capric acid, caproic acid, caprylic acid, laurylsulfuric acid, taurine, salicylic acid, myristic acid, palmitic acid, linolic acid, methasulfobenzoic acid, dioctylsulfosuccinic acid, tridecylsulfosuccinic acid, dibutylsulfosuccinic acid, dipalmitoyl phosphatidic acid, cinnamic acid, pentylsulfuric acid, heptylsulfuric acid, octylsulfuric acid, nonylsulfuric acid, 5-sulfoisophthalic acid, hexadecylsulfuric acid, tetradecylsulfuric acid, decylsulfuric acid, dodecylsulfuric acid, tetradecylsulfuric acid, lauric acid, ethylsulfuric acid, laurylphosphoric acid and 3-sulfobenzoic acid (preferably salicylic acid, deoxycholic acid, myristic acid, dodecylsulfuric acid, decylsulfuric acid, tetradecylsulfuric acid, lauric acid etc.). Preferable examples of the salt with a basic amino acid include salts with arginine, lysine and ornithine, and preferable examples of the salt with an acidic amino acid include salts with aspartic acid, glutamic acid etc.

In particular, salts between the water-insoluble or slightly soluble compound having CCR5 antagonism and salicylic acid, deoxycholic acid, myristic acid, dodecylsulfuric acid, decylsulfuric acid, tetradecylsulfuric acid, or lauric acid are preferably used, among which salts between N,N-dimethyl-N-(4-(((2-(4-methylphenyl)-6,7-dihydro-5H-benzocyclohepten-8-yl) carbonyl) amino) benzyl)-N-(4-tetrahydropyranyl) ammonium and anions derived from salicylic acid, deoxycholic acid, myristic acid, dodecylsulfuric acid, decylsulfuric acid, tetradecylsulfuric acid or lauric acid are preferable. Such salts can be produced in a method known in the art by salt exchange using N,N-dimethyl-N-(4-(((2-(4-methylphenyl)-6,7-dihydro-5H-benzocyclohepten-8-yl) carbonyl)amino)benzyl)-N-(4-tetrahydropyranyl) ammonium chloride and organic acids such as salicylic acid, deoxycholic acid, myristic acid, dodecylsulfuric acid, decylsulfuric acid, tetradecylsulfuric acid and lauric acid or salts thereof.

The "compound having CCR5 antagonism" used in this invention may be either a hydrate or non-hydrate. When the "compound having CCR5 antagonism" used in this invention occurs as a configurational isomer, a diastereomer and a conformer, these can be separated if necessary by separation and purification means known in the art. Further, when the "compound having CCR5 antagonism" used in this invention is a racemate, the racemate can be resolved by usual optical resolution means into (S)- and (R)-enantiomers, and both the optically active enantiomers and the racemate fall under the scope of this invention.

A prodrug of the "compound having CCR5 antagonism" or its salt [also referred to hereinafter as CCR5 antagonist] used in this invention refers to a compound to be converted into the CCR5 antagonist in vivo by reaction with an enzyme or gastric acid under physiological conditions, i.e. to a compound to be converted into the CCR5 antagonist by enzymatic oxidization, reduction or hydrolysis, or to a compound to be converted into the CCR5 antagonist by hydrolysis with gastric acid, etc. The prodrug of the CCR5 antagonist include those compounds of CCR5 antagonist whose amino group has been acylated, alkylated or phosphorylated (e.g., those compounds of CCR5 antagonist whose amino group has been eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, or tert-butylated; those compounds of CCR5 antagonist whose hydroxyl group has been acylated, alkylated, phosphorylated or borated (e.g., those compounds of CCR5 antagonist whose hydroxyl group has been acetylated, palmitoylated, propanoylated, pivaloylated, succinylated, fumarylated, alanylated or dimethylaminomethylcarbonylated); and those compounds of CCR5 antagonist whose carboxyl group has been esterified or amidated (e.g., those compounds of CCR5 antagonist whose carboxyl group has been ethylesterified, phenylesterified, carboxymethylesterified, dimethylaminomethylesterified, pivaloyoxyloxymethylesterified, ethoxycarbonyloxyethylesterified, phthalidylesterified, (5-methyl-2-oxo-1,3-dioxolen-4-yl)methylesterified, cyclohexyloxycarbonylethylesterified, or methylamidated). These compounds can be produced from the CCR5 antagonist by a method known in the art.

The prodrug of CCR5 antagonist may be a compound to be converted into the CCR5 antagonist under physiological conditions as described in "Iyakuhin No Kaihatsu" (Development of Pharmaceutical Preparations), vol. 7, "Bunshi Sekkei" (Molecular Design), pp. 163-198, published in 1990 by Hirokawa Shoten.

Further, the CCR5 antagonist may be labeled with isotopes (e.g., ³H, ¹⁴C, ³⁵S, ¹²⁵I etc.).

In this specification, the term "water-insoluble or slightly soluble" means that the amount of physiological saline necessary for dissolving 1 g solute within 30 minutes by stirring at 20 ±5°C for 5 minutes is 100 ml or more, preferably 1000 ml or more and more preferably 10000 ml or more.

The constituent components in a carrier used for (1) dissolution or (2) dispersion of the water-insoluble or slightly soluble compound having CCR5 antagonism or salts thereof may be any ones capable of dispersion or dissolution of the water-insoluble or slightly soluble compound having CCR5 antagonism or salts thereof, among which alcohols, amphipathic substances and lipids are preferably used. Dispersion used herein means that the compound has formed a uniform phase without being dissolved, and preferably the compound is homogenously dispersed particularly in a uniform state of fine particles, particularly preferably in the form of a uniform dispersion liquid at the molecular level.

The carrier may be a suitable combination of such components, and specific examples include a combination of at least one amphipathic substance having high HLB and at least one amphipathic substance having low HLB, a combination of at least one alcohol and at least one lipid, a combination of at least one amphipathic substance and at least one lipid, a combination of at least one amphipathic substance and at least one alcohol, a combination of at least one amphipathic substance, at least one alcohol and at least one lipid, a combination of at least one amphipathic substance having high HLB, at least one amphipathic substance having low HLB and a lipid, a combination of at least one amphipathic substance having high HLB, at least one amphipathic substance having low HLB and an alcohol, and two or more amphipathic substances, alcohols and lipids may be used as the constituent components in the carrier.

The alcohols used in this invention include, for example, propylene glycol, ethanol, glycerin, benzyl alcohol, polyethylene glycol 200, 300 or 400, polyoxyethylene castor oil derivatives, polyoxyethylene stearate, lauryl alcohol, palmitoyl alcohol, stearyl alcohol, diethylene glycol monoethyl ether (TRANSCUTOL, Gatefosse Ltd.) (preferably polypropylene glycol, lauryl alcohol and diethylene glycol monoethyl ether).

The amphipathic substances used in this invention include sorbitan fatty acid esters such as sorbitan monolaurate, sorbitan monopalmitinate, sorbitan monostearate, sorbitan trioleate and sorbitan monooleate; saturated polyglycolized glycerides such as Gelucire 35/10, 44/14, 46/07, 50/13, 53/10, LABRASOL, LABRAFAC CH10 (Gattefosse Ltd.) (preferably Gelucire 44/14, LABRASOL etc.); polyglycerine fatty acid esters such as tetraglycerine monooleate (MO-310, Sakamoto Yakuhin), tetraglycerine monolaurate (ML-310, Sakamoto Yakuhin), tetraglycerine monocaprylate (MCA-310, Sakamoto Yakuhin), hexaglycerine monooleate (MO-500, Sakamoto Yakuhin), hexaglycerine monolaurate (ML-500, Sakamoto Yakuhin), hexaglycerine monocaprylate (MCA-500, Sakamoto Yakuhin), decaglycerine monooleate (MO-750, Sakamoto Yakuhin), decaglycerine monolaurate (ML-750, Sakamoto Yakuhin), decaglycerine monocaprylate (MCA-750, Sakamoto Yakuhin), tetraglycerine monostearate (MS-310, Sakamoto Yakuhin), hexaglycerine monostearate (MS-500, Sakamoto Yakuhin), hexaglycerine sesquistearate (SS-500, Sakamoto Yakuhin), decaglycerine monostearate (MS-750, Sakamoto Yakuhin), decaglycerine tristearate (TS-750, Sakamoto Yakuhin) and decaglycerine sesquioleate (SO-750, Sakamoto Yakuhin); polyoxyethylene polypropylene glycols such as Poloxame R⁶8; monofatty acid esters of polyoxyethylene (20) sorbitan such as Tween 20, 40, 60 and 80; unsaturated polyglycolized glycerides such as D-α-tocopheryl polyethylene glycol 1000 succinate (Vitamin E TPGS), LABRAFIL WL 2609 BS, LABRAFIL M 1944 CS, LABRAFIL M 2125 CS, LABRAFIL ISOSTEARIQUE, LAUROGLYCOL FCC, PLUROL OLEIQUE CC 497 (Gattefosse Ltd.) (preferably LABRAFIL WL 2609 BS etc.); and polyglycerine fatty acid esters such as laurylate propylene glycol (LAUROGLYCOL FCC) (Gatefosse Ltd.); PLUROL OLEIQUE CC 497 (Gatefosse Ltd.), tetraglycerine pentaoleate (PO-310), hexaglycerine pentaoleate (PO-500), hexaglycerine decaoleate (DAO-750), tetraglycerine tristearate (TS-310, Sakamoto Yakuhin), tetraglycerine pentastearate (PS-310, Sakamoto Yakuhin), hexaglycerine tristearate (TS-500, Sakamoto Yakuhin), hexaglycerine pentastearate (PS-500, Sakamoto Yakuhin), decaglycerine decastearate (DAS-750, Sakamoto Yakuhin) and decaglycerine heptabehenate (HB-750, Sakamoto Yakuhin); and Cremophor EL.

The amphipathic substances having high HLB include saturated polyglycolized glycerides such as Gelucire 35/10, 44/14, 46/07, 50/13, 53/10, LABRASOL, LABRAFAC CH10 (Gattefosse Ltd.); polyglycerine fatty acid esters such as tertaglycerine monooleate (MO-310, Sakamoto Yakuhin), tetraglycerine monolaurate (ML-310, Sakamoto Yakuhin), tetraglycerine monocaprylate (MCA-310, Sakamoto Yakuhin), hexaglycerine monooleate (MO-500, Sakamoto Yakuhin), hexaglycerine monolaurate (ML-500, Sakamoto Yakuhin), hexaglycerine monocaprylate (MCA-500, Sakamoto Yakuhin), decaglycerine monooleate (MO-750, Sakamoto Yakuhin), decaglycerine monolaurate (ML-750, Sakamoto Yakuhin), decaglycerine monocaprylate (MCA-750, Sakamoto Yakuhin), tetraglycerine monostearate (MS-310, Sakamoto Yakuhin), hexaglycerine monostearate (MS-500, Sakamoto Yakuhin), hexaglycerine sesquistearate (SS-500, Sakamoto Yakuhin), decaglycerine monostearate (MS-750, Sakamoto Yakuhin), decaglycerine tristearate (TS-750, Sakamoto Yakuhin) and decaglycerine sesquioleate (SO-750, Sakamoto Yakuhin); polyoxyethylene polypropylene glycols such as Poloxamer 68; monofatty acid esters of polyoxyethylene (20) sorbitan such as Tween 20, 40, 60 and 80; D-α-tocopheryl polyethylene glycol 1000 succinate (Vitamin E TPGS) (preferably Gelucire 44/14, LABRASOL, Vitamin E TPGS etc.).

The amphipathic substances having low HLB include unsaturated polyglycolized glycerides such as LABRAFIL WL 2609 BS, LABRAFIL M 1944 CS, LABRAFIL M 2125 CS, LABRAFIL ISOSTEARIQUE, LAUROGLYCOL FCC, PLUROL OLEIQUE CC 497 (Cattefosse Ltd.); laurate propylene glycol (LAUROGLYCOL FCC) (Gatefosse Ltd.); polyglycerine fatty acid esters such as PLUROL OLEIQUE CC 497 (Gatefosse Ltd.), tetraglycerine pentaoleate (PO-310, Sakamoto Yakuhin), hexaglycerine pentaoleate (PO-500, Sakamoto Yakuhin), decaglycerine decaoleate (DAO-750, Sakamoto Yakuhin), tetraglycerine tristearate (TS-310, Sakamoto Yakuhin), tetraglycerine pentastearate (PS-310, Sakamoto Yakuhin), hexaglycerine tristearate (TS-500, Sakamoto Yakuhin), hexaglycerine pentastearate (PS-500, Sakamoto Yakuhin), decaglycerine decastearate (DAS-750, Sakamoto Yakuhin), decaglycerine heptabehenate (HB-750, Sakamoto Yakuhin); and Cremophor EL (preferably LABRAFIL WL 2609 BS etc.).

In this specification, the amphipathic substances having high HLB mean amphipathic substances having an HLB of 8 or more, while the amphipathic substances having low HLB mean amphipathic substances having an HLB of less than 8.

The amphipathic substances used in this invention are preferably a combination of one or more amphipathic substances (e.g. a combination of LABRASOL and LABRAFIL WL 2609 BS) selected from saturated polyglycolized glycerides (preferably Gelucire 44/14, LABRASOL etc.), unsaturated polyglycolized glycerides (preferably LABRAFIL WL 2609 BS etc.) and D-α-tocopheryl polyethylene glycol 1000 succinate.

The lipids used in this invention include fats and oils such as LABRAFAC LIPOPHILE, LABRAFAC CC, Gelucire 33/01, Gelucire 39/01, Gelucire 43/01, LABRAFAC PG, olive oil, sesame oil, soybean oil, corn oil, rapeseed oil, castor oil, coconut oil and eucalyptus oil; middle-chain fatty acid triglycerides such as Miglyol 812; triglycerides such as tricaprylin, trilaurin; polyglycerine fatty acid esters such as tetraglycerine polyricinoleate (CR-310, Sakamoto Yakuhin), hexaglycerine polyricinoleate (CR⁵00, Sakamoto Yakuhin), condensed polyricinoleate (CR-ED, CRS-75, Sakamoto Yakuhin), tetraglycerine-mixed fatty acid esters (THL-3, THL-15, Sakamoto Yakuhin), as well as Sefsol 228 and egg yolk lecithin.

The pharmaceutical composition for oral use in this invention may be a liquid, a semisolid (for example, an intermediate state between a liquid such as paste and a solid or a state of highly viscous liquid) or a solid at ordinary temperature (15 to 25 °C), more preferably a liquid or semisolid.

The content of the compound having CCR5 antagonism or salts thereof in the composition of this invention is 0.01 to 90 w/w-%, preferably 0.1 to 50 w/w-%, more preferably 1 to 30 w/w-%.

The administration form is not particularly limited insofar as the active ingredient can be orally administered, and examples thereof include liquids, elixirs, capsules, granules, suspensions or emulsions, powders, tablets and syrups, among which capsules are preferably used.

The carrier used in this invention may be composed exclusively of the above-described constituent components, but may be blended if necessary with pharmaceutically acceptable additives. For example, additives suitably added for promotion of oral absorption of the composition of this invention (oral absorption promoting additives) include alkali metal salts (e.g., sodium salts, potassium salts etc.), alkaline earth metal salts (e.g., potassium salts, magnesium salts etc.) with organic acids such as acetic acid, propionic acid, capric acid, stearic acid, sorbic acid, oleic acid, benzoic acid, lactic acid, gluconic acid, dehydroacetic acid, erysorbic acid, deoxycholic acid, cholic acid, laurylsulfuric acid, taurine, salicylic acid, myristic acid, palmitic acid, linolic acid, methasulfobenzoic acid, dioctylsulfosuccinic acid, tridecylsulfosuccinic acid, dibutylsulfosuccinic acid, dipalmitoyl phosphatidic acid, cinnamic acid, pentylsulfuric acid, heptylsulfuric acid, octylsulfuric acid, nonylsulfuric acid, 5-sulfoisophthalic acid, hexadecylsulfuric acid, tetradecylsulfuric acid, decylsulfuric acid, dodecylsulfuric acid, tetradecylsulfuric acid, ethylsulfuric acid, laurylphosphoric acid, 3-sulfobenzoic acid, lauric acid, caprylic acid, caproic acid and caprylic acid (preferably, salicylic acid, deoxycholic acid, myristic acid, dodecylsulfuric acid, decylsulfuric acid, tetradecylsulfuric acid, lauric acid etc.), among which sodium salicylate, sodium deoxycholate, sodium myristate, sodium dodecylsulfate, sodium decylsulfate, sodium tetradecylsulfate and sodium laurate are preferably used as absorption promoting additives.

The pharmaceutically acceptable additives used in this invention are conventional organic or inorganic carrier materials in pharmaceutical preparations, and blended as solvents, solubilizers and suspending agents. If necessary, pharmaceutical additives such as preservatives, antioxidants, coloring agents and sweeteners can also be used.

Preferable examples of the solvents include injection water, alcohol, propylene glycol, macrogol, sesame oil, corn oil etc. Preferable examples of the solubilizers include polyethylene glycol, propylene glycol, D-mannitol, benzyl benzoate, ethanol, trisaminomethane, cholesterol, triethanolamine, sodium carbonate, sodium citrate, etc. Preferable examples of the suspending agents include surfactants such as stearyl triethanolamine, sodium laurylsulfate, lauryl aminopropionic acid, lecithin, benzalconium chloride, benzetonium chloride, and glycerine monostearate; hydrophilic polymers such as polyvinyl alcohol, polyvinyl pyrrolidone, sodium carboxymethyl cellulose, methyl cellulose, hydroxymethyl cellulose, hydroxyethyl cellulose, hydroxypropyl cellulose, and polyacrylic acid derivatives or salts thereof (e.g. carboxyvinyl polymers). Preferable examples of the preservatives include paraoxybenzoates, chlorobutanol, benzyl alcohol, phenetyl alcohol, hydroacetic acid, and sorbic acid. Preferable examples of the antioxidants include sulfites, ascorbic acid, tocopherol etc.

As emulsion stabilizers, it is possible to suitably use naturally occurring stabilizers such as carrageenan, alginic acid, phaselan [phonetic trans.], locust bean gum, guar gum, tamarind gum, Arabia gum, tragacanth gum, karaya gum, pectin, arabinogalactan, xanthane gum, pullulan, crystalline cellulose, casein, gelatin, plant protein hydrolyzates, processed starch and dextrin, and synthetic stabilizers such as sodium alginate, alginic acid PG ester, cellulose calcium glycolate, cellulose sodium glycolate, methyl cellulose, casein sodium, starch sodium phosphate, processed starch, starch sodium glycolate, and sodium polyacrylate.

The pharmaceutical composition for oral use according to this invention can be produced by dissolving or dispersing the water-insoluble or slightly soluble compound having CCR5 antagonism or its salt (preferably in a uniform dispersion liquid of fine particles) in a carrier containing at least one constituent component selected from amphipathic substances, alcohols and lipids, and an emulsion formed by suitably adding water thereto can also be used. In this case, water added may be contained in the system of either the water-insoluble or slightly soluble compound having CCR5 antagonism (or its salt) or a carrier, and a carrier containing an amphipathic substance (preferably a self-emulsifiable surfactant) is preferably used.

Further, the composition of this invention can be used by being adsorbed into or mixed with pharmaceutically acceptable powdery additives. Such powdery additives include e.g. lactate, crystalline cellulose, titanium oxide, talc, synthetic aluminum silicate, croscarmellose sodium, starch, calcium carbonate, low-substituted hydroxypropyl cellulose, carboxymethyl starch sodium, crospopidone [phonetic trans.] etc. The ratio of these additives to the powder composition is about 0.01 to 100 w/w%, preferably about 0.1 to 10 w/w%, more preferably about 1 to 3 w/w%.

The pharmaceutical composition for oral use according to this invention can be produced for example by adding the water-insoluble or slightly soluble compound having CCR5 antagonism or salts thereof to a carrier containing at least one constituent component selected from amphipathic substances, alcohols and lipids (if necessary in a liquid state by heating) and then stirring it with a stirrer or an emulsifying machine such as a propeller stirrer, a homomixer, a sonicator, a vortex stirrer, a galling homogenizer or a micro-fluidizer generally used in production of foods or pharmaceutical preparation thereby dissolving or dispersing the water-insoluble or slightly soluble compound having CCR5 antagonism or salts thereof (preferably in a uniform dispersion liquid of fine particles). Further, the pharmaceutical preparation for oral use can be charged into hard or soft capsules such as gelatin capsules and HPMC capsules by a general liquid or semisolid capsule-charging machine, to prepare capsules.

Further, the pharmaceutical preparation for oral use can also be produced by dissolving or dispersing the water-insoluble or slightly soluble compound having CCR5 antagonism or salts thereof (preferably in a uniform dispersion liquid of fine particles) in a carrier melted at a temperature higher than the melting point thereof (preferably a temperature higher by at least 10 °C than the melting point, more preferably a temperature higher by at least 20 °C than the melting point) followed by cooling thereof. Alternatively, the pharmaceutical preparation for oral use according to this invention can be produced from a dispersion of the water-insoluble or slightly soluble compound having CCR5 antagonism or salts thereof in water or an aqueous solution thereof prepared by dissolution under heating and a carrier containing at least one constituent component selected from amphipathic substances, alcohols and lipids.

The "compound having CCR5 antagonism" or salts thereof used in this invention may be used in combination with other agents for preventing and treating HIV infections (in particular agents for preventing and treating AIDS).

The other agents for preventing and treating HIV infections, which are used in combination with the "compound having CCR5 antagonism" in this invention, include e.g. nucleic acid type reverse transcriptase inhibitors such as zidovudine, didanosine, zalcitabine, lamivudine, stavudine, abacavir, adefovir, adefovir dipivoxil, and fozivudine tidoxil; non-nucleic acid type reverse transcriptase inhibitors such as nevirapine, delavirdine, efavirenz, loviride, immunocal and oltipraz (including drugs having antioxidant action, such as immunocal and oltipraz); and protease inhibitors such as saquinavir, ritonavir, indinavir, nelfinavir, amprenavir, palinavir and lasinavir.

The nucleic acid-type reverse transcriptase inhibitors are preferably zidovudine, didanosine, zalcitabine, lamivudine, stavudine and abacavir; the non-nucleic acid-type reverse transcriptase inhibitors are preferably nevirapine, delavirdine and efavirenz; and the protease inhibitors are preferably saquinavir, ritonavir, indinavir, nelfinavir and amprenavir.

The "compound having CCRS antagonism" or salts thereof used in this invention can be used in combination with not only the above-described protease inhibitors and nucleic acid-type reverse transcriptase inhibitors but also CXCR4 antagonists (e.g., AMD-3100 etc.) as second receptors for T cell-targeting HIV-1, antibodies against surface antigens of HIV-1, and HIV-1 vaccines.

The compounds represented by formula (I) or (Ia) or salts thereof can be produced in a method known in the art. For example, these can be produced by a method described in JP-A 8-73476 or a modified method thereof, or by a method described in WO99/32100 and WO99/32468 or a modified method thereof.

The daily dose of the "compound having CCR5 antagonism" or salts thereof used in this invention is varied depending on the condition and weight of a patient and an administration method, and for example in the case of oral administration, about 5 to 1000 mg, preferably about 10 to 600 mg, more preferably about 10 to 300 mg and most preferably about 15 to 150 mg active ingredient [CCR5 antagonist] is administered daily into an adult (weighing about 50 kg) once or in two or three divided portions.

Further, the CCR5 antagonist is used in combination with a reverse transcriptase inhibitor and/or a protease inhibitor, the dose of the reverse transcriptase inhibitor or protease inhibitor is selected in the range of from about 1/200 or 1/2 to about 2- or 3-fold relative to the usual dose. Further, when the CCR5 antagonist is used in combination with two or more drugs, the dose of each drug administered is suitably regulated if one of the drugs influences the metabolism of other drugs, but generally each drug is used in a dose used in single administration of the drug.

### BEST MODE FOR CARRYING OUT THE INVENTION

Hereinafter, this invention is described in more detail by reference to the Examples and Experimental Examples. However, these examples are described for illustrative purposes and are not intended to limit the present invention.

### EXAMPLE 1

N,N-dimethyl-N-(4-(((2-(4-methylphenyl)-6,7-dihydro-5H-benzocyclohepten-8-yl)carbonyl)amino)benzyl)-N-(4-tetrahydropyranyl)ammonium chloride [referred to hereinafter as Compound A] (3 mg) was added under stirring to Gelucire 44/14 (1 g) melted at a temperature higher by at least 20 °C than the melting point thereof, and the mixture was sufficiently stirred to prepare a uniform dispersion liquid of fine particles, which was then cooled to room temperature.

### EXAMPLE 2

Compound A (3 mg) was added under stirring to Gelucire 50/13 (1 g) melted at a temperature higher by at least 20 °C than the melting point thereof, and the mixture was sufficiently stirred to prepare a uniform dispersion liquid of fine particles, which was then cooled to room temperature.

### EXAMPLE 3

Compound A (3 mg) was added under stirring to Gelucire 53/10 (1 g) melted at a temperature higher by at least 20 °C than the melting point thereof, and the mixture was sufficiently stirred to prepare a uniform dispersion liquid of fine particles, which was then cooled to room temperature.

### EXAMPLE 4

Compound A (3 mg) was added under stirring to Vitamin E TPGS (1 g) melted at a temperature higher by at least 20 °C than the melting point thereof, and the mixture was sufficiently stirred to prepare a transparent solution, which was then cooled to room temperature.

### EXAMPLE 5

Compound A (3 mg) was added under stirring to decaglycerine monolaurate (ML-750) (1 g) melted at a temperature higher by at least 20 °C than the melting point thereof, and the mixture was sufficiently stirred to prepare a transparent solution, which was then cooled to room temperature.

### EXAMPLE 6

Compound A (3 mg) was added under stirring to decaglycerine monocaprylate (MCA-750) (1 g) melted at a temperature higher by at least 20 °C than the melting point thereof, and the mixture was sufficiently stirred to prepare a uniform dispersion liquid of fine particles, which was then cooled to room temperature.

### EXAMPLE 7

Compound A (3 mg) was added under stirring to decaglycerine monooleate (MO-750) (1 g) melted at a temperature higher by at least 20 °C than the melting point thereof, and the mixture was sufficiently stirred to prepare a uniform dispersion liquid of fine particles, which was then cooled to room temperature.

### EXAMPLE 8

Compound A (3 mg) was added under stirring to decaglycerine decaoleate (DAO-750) (1 g) melted at a temperature higher by at least 20 °C than the melting point thereof, and the mixture was sufficiently stirred to prepare a uniform dispersion liquid of fine particles, which was then cooled to room temperature.

### EXAMPLE 9

Compound A (3 mg) was added under stirring to hexaglycerine monolaurate (ML-500) (1 g) melted at a temperature higher by at least 20 °C than the melting point thereof, and the mixture was sufficiently stirred to prepare a transparent solution, which was then cooled to room temperature.

### EXAMPLE 10

The preparations prepared in Examples 4, 5 and 9 were charged into gelatin capsules to prepare capsules.

### EXAMPLE 11

The preparations prepared in Example 4, 5 and 9 were charged into HPMC capsules to prepare capsules.

### EXAMPLE 12

The preparations prepared in Example 4, 5 and 9 were charged into enteric capsules (enteric AS capsules) to prepare capsules.

### EXAMPLE 13

LABRAFIL WL 2609 BS (400 mg) and TRANSCUTOL (150 mg) were added to LABRASOL (450 mg) under stirring to prepare a transparent mixed solution. Compound A (3 mg) was added to this solution and sufficiently stirred to prepare a uniform dispersion liquid of fine particles.

### EXAMPLE 14

LABRAFIL WL 2609 BS (400 mg) and PEG400 (150 mg) were added to LABRASOL (450 mg) under stirring to prepare a transparent mixed solution. Compound A (3 mg) was added to this solution and sufficiently stirred to prepare a uniform dispersion liquid of fine particles.

### EXAMPLE 15

LABRAFIL WL 2609 BS (400 mg) and TRANSCUTOL (150 mg) were added at 90 °C to LABRASOL (450 mg) under stirring to prepare a transparent mixed solution. Compound A (3 mg) was added to this solution and sufficiently stirred to prepare a uniform dispersion liquid of fine particles, which was then cooled to room temperature.

### EXAMPLE 16

DAO-750 (400 mg) and TRANSCUTOL (150 mg) were added at 90 °C to ML-750 (450 mg) under stirring to prepare a transparent mixed solution. Compound A (3 mg) was added to this solution and sufficiently stirred to prepare a transparent mixed solution, which was then cooled to room temperature.

### EXAMPLE 17

LABRAFIL WL 2609 BS (400 mg) and TRANSCUTOL (150 mg) were added at 90 °C to Vitamin E TPGS (450 mg) under stirring to prepare a transparent mixed solution. Compound A (3 mg) was added to this solution and sufficiently stirred to prepare a transparent mixed solution, which was then cooled to room temperature.

### EXAMPLE 18

LABRAFIL WL 2609 BS (400 mg) and TRANSCUTOL (150 mg) were added to LABRASOL (450 mg) under stirring to prepare a transparent mixed solution. Compound A (10 mg) was added to this solution and sufficiently stirred to prepare a uniform dispersion liquid of fine particles.

### EXAMPLE 19

Gelucire 44/14 (690 mg) was melted at a temperature higher by at least 20 °C than the melting point thereof. A dispersion of Compound A (10 mg) in propylene glycol (300 mg) was added to this solution and sufficiently stirred to prepare a uniform dispersion liquid of fine particles, which was then cooled to room temperature.

### EXAMPLE 20

LABRAFIL WL 2609 BS (360 mg) and TRANSCUTOL (100 mg) were added to LABRASOL (430 mg) under stirring to prepare a transparent mixed solution. A dispersion of Compound A (10 mg) in propylene glycol (100 mg) was added to this solution and sufficiently stirred to prepare a uniform dispersion liquid of fine particles.

### EXAMPLE 21

Compound A (10 mg) was dissolved by heating in purified water (0.5 ml) and then cooled to room temperature. After an aqueous solution (0.5 ml) prepared by dissolving sodium lauryl sulfate in a 2-fold molar amount relative to Compound A was added thereto and sufficiently stirred, Sefsol 228 (300 mg) was added thereto and sufficiently stirred. LABRASOL/LABRAFIL WL 2609 BS (9/8) solution (1700 mg) was added to the mixture and sufficiently stirred to prepare a transparent solution.

### EXAMPLE 22

Compound A (10 mg) was dissolved by heating in purified water (0.5 ml) and then cooled to room temperature. After an aqueous solution (0.5 ml) prepared by dissolving sodium acetate in a 2-fold molar amount relative to Compound A was added thereto and then sufficiently stirred, Sefsol 228 (300 mg) was added thereto and sufficiently stirred. LABRASOL/LABRAFIL WL 2609 BS (9/8) solution (1700 mg) was added thereto and sufficiently stirred to make a transparent solution.

### EXAMPLE 23

Compound A (10 mg) was dissolved by heating in purified water (0.5 ml) and then cooled to room temperature. After an aqueous solution (0.5 ml) prepared by dissolving taurine in a 2-fold molar amount relative to Compound A was added thereto and then sufficiently stirred, Sefsol 228 (300 mg) was added thereto and sufficiently stirred. LABRASOL/LABRAFIL WL 2609 BS (9/8) solution (1700 mg) was added thereto and sufficiently stirred to make a transparent solution.

### EXAMPLE 24

Compound A (10 mg) was dissolved by heating in purified water (0.5 ml) and then cooled to room temperature. After an aqueous solution (0.5 ml) prepared by dissolving sodium benzoate in a 2-fold molar amount relative to Compound A was added thereto and then sufficiently stirred, Sefsol 228 (300 mg) was added thereto, sufficiently stirred and then left. LABRASOL/LABRAFIL WL 2609 BS (9/8) solution (1700 mg) was added to the oil phase (Sefsol phase) and sufficiently stirred to make a transparent solution.

### EXAMPLE 25

Egg yolk lecithin (16 mg) and Cremophor EL (166 mg) were added to Sefsol 228 (765 mg) under stirring to prepare a transparent solution. A suspension of Compound A (2 mg) in purified water (53 mg) was added gradually under stirring to this solution to prepare a uniform dispersion liquid of fine particles.

### EXAMPLE 26

Egg yolk lecithin (16 mg) and Cremophor EL (166 mg) were added to Sefsol 228 (765 mg) under stirring to prepare a transparent solution. A solution prepared by dissolving Compound A (2 mg) in purified water (53 mg) under heating and then cooling the solution to room temperature was added gradually to this solution and sufficiently stirred to prepare a transparent solution.

### EXAMPLE 27

Polyglycerine-condensed ricinoleate ester (CRS-75) (80 mg) was added to Sefsol 228 (720 mg) under stirring to prepare a transparent solution. A suspension of Compound A (2 mg) in glycerine (80 mg) was gradually added to the solution under stirring, to prepare a uniform dispersion liquid of fine particles.

### EXAMPLE 28

The preparations prepared in Examples 13, 15, 16 and 17 were charged into gelatin capsules to prepare capsules.

### EXAMPLE 29

The preparations prepared in Example 13, 15, 16 and 17 were charged into HPMC capsules to prepare capsules.

### EXAMPLE 30

The preparations prepared in 13, 15, 16 and 17 were charged into enteric capsules (enteric AS capsules) to prepare capsules.

### EXPERIMENTAL EXAMPLE 1

A pharmaceutical preparation prepared in each of Examples 13, 15, 25 and 26 was administered into the duodena of rats under anesthesia which had previously been fasted for 24 hours. 0.5, 1, 2 and 4 hours after the administration, blood was collected via carotid arteries from the rats. Plasma samples obtained by centrifugation were measured for the concentration of Compound A therein, and AUC for 4 hours after the administration was determined by a trapezoid rule. As the control, a suspension containing Compound A was administered similarly into duodena, and AUC for 4 hours after the administration was similarly determined. As a result, the absorption was significantly increased by administering the preparations of the invention than by administering the suspension, as shown in Table 1.

**Table 1**

| Preparations | AUC (µg × hr/ml) |
|---|---|
| Control (suspension) | 0.007 |
| Example 13 | 0.325 |
| Example 15 | 0.308 |
| Example 25 | 0.169 |
| Example 26 | 0.139 |

### EXAMPLE 31

Compound A (50 mg) was added under stirring to decaglycerine monolaurate (ML-750) (500 mg) melted at a temperature higher by at least 20°C than the melting point thereof, and the mixture was sufficiently stirred to prepare a uniform dispersion liquid of fine particles, which was then cooled to room temperature.

### EXAMPLE 32

Compound A (50 mg) was added under stirring to hexaglycerine monolaurate (ML-500) (500 mg) melted at a temperature higher by at least 20 °C than the melting point thereof, and the mixture was sufficiently stirred to prepare a uniform dispersion liquid of fine particles, which was then cooled to room temperature.

### EXAMPLE 33

PO-500 (400 mg) and TRANSCUTOL (150 mg) were added to ML-750 (450 mg) at 90 °C under stirring to prepare a transparent mixed solution. Compound A (100 mg) was added to this solution and sufficiently stirred to prepare a uniform dispersion liquid of fine particles, which was then cooled to room temperature.

### EXAMPLE 34

PO-500 (400 mg) and Sefsol 228 (150 mg) were added to ML-750 (450 mg) at 90 °C under stirring to prepare a transparent mixed solution. Compound A (100 mg) was added to this solution and sufficiently stirred to prepare a uniform dispersion liquid of fine particles, which was then cooled to room temperature.

### EXAMPLE 35

TRANSCUTOL (125 mg) was added to ML-750 (375 mg) at 90 °C under stirring to prepare a transparent solution. Compound A (50 mg) was added to this solution and sufficiently stirred to prepare a uniform dispersion liquid of fine particles, which was then cooled to room temperature.

### Example 36

Compound A (5 mg) was dissolved by heating in purified water (0.1 mL) and then cooled to room temperature. An aqueous solution (0.25 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in propylene glycol (100 mg) and cooled to room temperature.

### Example 37

Compound A (5 mg) was dissolved by heating in purified water (0.1 mL) and then cooled to room temperature. An aqueous solution (0.25 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in polyethylene glycol 400 (100 mg) and cooled to room temperature.

### Example 38

Compound A (5 mg) was dissolved by heating in purified water (0.1 mL) and then cooled to room temperature. An aqueous solution (0.25 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in tetraglycerine monolaurate (100 mg) and cooled to room temperature.

### Example 39

Compound A (5 mg) was dissolved by heating in purified water (0.1 mL) and then cooled to room temperature. An aqueous solution (0.25 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in tetraglycerine monooleate (100 mg) and cooled to room temperature.

### Example 40

Compound A (5 mg) was dissolved by heating in purified water (0.1 mL) and then cooled to room temperature. An aqueous solution (0.25 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in sorbitan monooleate (100 mg) and cooled to room temperature.

### Example 41

Compound A (5 mg) was dissolved by heating in purified water (0.1 mL) and then cooled to room temperature. An aqueous solution (0.25 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in lauryl alcohol (100 mg) and cooled to room temperature.

### Example 42

Compound A (5 mg) was dissolved by heating in purified water (0.1 mL) and then cooled to room temperature. An aqueous solution (0.25 mL) prepared by dissolving sodium deoxycholate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in propylene glycol (100 mg) and cooled to room temperature.

### Example 43

Compound A (5 mg) was dissolved by heating in purified water (0.1 mL) and then cooled to room temperature. An aqueous solution (0.25 mL) prepared by dissolving sodium deoxycholate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in polyethylene glycol 400 (100 mg) and cooled to room temperature.

### Example 44

Compound A (5 mg) was dissolved by heating in purified water (0.1 mL) and then cooled to room temperature. An aqueous solution (0.25 mL) prepared by dissolving sodium deoxycholate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in tetraglycerine monolaurate (100 mg) and cooled to room temperature.

### Example 45

Compound A (5 mg) was dissolved by heating in purified water (0.1 mL) and then cooled to room temperature. An aqueous solution (0.25 mL) prepared by dissolving sodium deoxycholate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in tetraglycerine monooleate (100 mg) and cooled to room temperature.

### Example 46

Compound A (5 mg) was dissolved by heating in purified water (0.1 mL) and then cooled to room temperature. An aqueous solution (0.25 mL) prepared by dissolving sodium deoxycholate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in sorbitan monooleate (100 mg) and cooled to room temperature.

### Example 47

Compound A (5 mg) was dissolved by heating in purified water (0.1 mL) and then cooled to room temperature. An aqueous solution (0.25 mL) prepared by dissolving sodium deoxycholate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in lauryl alcohol (100 mg) and cooled to room temperature.

### Example 48

Compound A (5 mg) was dissolved by heating in purified water (0.1 mL) and then cooled to room temperature. An aqueous solution (0.25 mL) prepared by dissolving sodium myristate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in propylene glycol (100 mg) and cooled to room temperature.

### Example 49

Compound A (5 mg) was dissolved by heating in purified water (0.1 mL) and then cooled to room temperature. An aqueous solution (0.25 mL) prepared by dissolving sodium myristate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in polyethylene glycol 400 (100 mg) and cooled to room temperature.

### Example 50

Compound A (5 mg) was dissolved by heating in purified water (0.1 mL) and then cooled to room temperature. An aqueous solution (0.25 mL) prepared by dissolving sodium myristate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in tetraglycerine monolaurate (100 mg) and cooled to room temperature.

### Example 51

Compound A (5 mg) was dissolved by heating in purified water (0.1 mL) and then cooled to room temperature. An aqueous solution (0.25 mL) prepared by dissolving sodium myristate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in tetraglycerine monooleate (100 mg) and cooled to room temperature.

### Example 52

Compound A (5 mg) was dissolved by heating in purified water (0.1 mL) and then cooled to room temperature. An aqueous solution (0.25 mL) prepared by dissolving sodium myristate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in sorbitan monooleate (100 mg) and cooled to room temperature.

### Example 53

Compound A (5 mg) was dissolved by heating in purified water (0.1 mL) and then cooled to room temperature. An aqueous solution (0.25 mL) prepared by dissolving sodium myristate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in lauryl alcohol (100 mg) and cooled to room temperature.

### Example 54

Compound A (45 mg) was dissolved by heating in purified water (0.9 mL) and then cooled to room temperature. An aqueous solution (0.9 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in propylene glycol (1450 mg) and cooled to room temperature.

### Example 55

Compound A (45 mg) was dissolved by heating in purified water (0.9 mL) and then cooled to room temperature. An aqueous solution (0.9 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in lauryl alcohol (1450 mg) and cooled to room temperature.

### Example 56

Compound A (45 mg) was dissolved by heating in purified water (0.9 mL) and then cooled to room temperature. An aqueous solution (0.9 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in sorbitan monooleate (1450 mg) and cooled to room temperature.

### Example 57

Compound A (45 mg) was dissolved by heating in purified water (0.9 mL) and then cooled to room temperature. An aqueous solution (0.9 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in tetraglycerine monooleate (1450 mg) and cooled to room temperature.

### Example 58

Compound A (45 mg) was dissolved by heating in purified water (0.9 mL) and then cooled to room temperature. An aqueous solution (0.9 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in Vitamin E TPGS (1450 mg) and cooled to room temperature.

### Example 59

Compound A (45 mg) was dissolved by heating in purified water (0.9 mL) and then cooled to room temperature. An aqueous solution (0.9 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in Gelucire 44/14 (1450 mg) and cooled to room temperature.

### Example 60

Compound A (45 mg) was dissolved by heating in purified water (0.9 mL) and then cooled to room temperature. An aqueous solution (0.9 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in TRANSCUTOL (217.5 mg) and cooled to room temperature. Further, a solution (1232.5 mg) of LABRASOL/LABRAFIL WL 2609 BS (9/8) was added thereto to prepare a mixed solution.

### Example 61

Compound A (45 mg) was dissolved by heating in purified water (0.9 mL) and then cooled to room temperature. An aqueous solution (0.9 mL) prepared by dissolving sodium deoxycholate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in propylene glycol (1450 mg) and cooled to room temperature.

### Example 62

Compound A (45 mg) was dissolved by heating in purified water (0.9 mL) and then cooled to room temperature. An aqueous solution (0.9 mL) prepared by dissolving sodium deoxycholate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in lauryl alcohol (1450 mg) and cooled to room temperature.

### Example 63 (1)

Compound A (45 mg) was dissolved by heating in purified water (0.9 mL) and then cooled to room temperature. An aqueous solution (0.9 mL) prepared by dissolving sodium deoxycholate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in sorbitan monooleate (1450 mg) and cooled to room temperature.

### Example 63 (2)

Compound A (45 mg) was dissolved by heating in purified water (0.9 mL) and then cooled to room temperature. An aqueous solution (0.9 mL) prepared by dissolving sodium deoxycholate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in tetraglycerine monooleate (1450 mg) and cooled to room temperature.

### Example 64

Compound A (45 mg) was dissolved by heating in purified water (0.9 mL) and then cooled to room temperature. An aqueous solution (0.9 mL) prepared by dissolving sodium deoxycholate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in Vitamin E TPGS (1450 mg) and cooled to room temperature.

### Example 65

Compound A (45 mg) was dissolved by heating in purified water (0.9 mL) and then cooled to room temperature. An aqueous solution (0.9 mL) prepared by dissolving sodium deoxycholate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in Gelucire 44/14 (1450 mg) and cooled to room temperature.

### Example 66

Compound A (45 mg) was dissolved by heating in purified water (0.9 mL) and then cooled to room temperature. An aqueous solution (0.9 mL) prepared by dissolving sodium deoxycholate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in TRANSCUTOL (217.5 mg) and cooled to room temperature. Further, a solution of LABRASOL/LABRAFIL WL 2609 BS (9/8) (1232.5 mg) was added to this mixture, to prepare a mixed solution.

### Example 67

Compound A (45 mg) was dissolved by heating in purified water (0.9 mL) and then cooled to room temperature. An aqueous solution (0.9 mL) prepared by dissolving sodium myristate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in propylene glycol (1450 mg) and cooled to room temperature.

### Example 68

Compound A (45 mg) was dissolved by heating in purified water (0.9 mL) and then cooled to room temperature. An aqueous solution (0.9 mL) prepared by dissolving sodium myristate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in lauryl alcohol (1450 mg) and cooled to room temperature.

### Example 69

Compound A (45 mg) was dissolved by heating in purified water (0.9 mL) and then cooled to room temperature. An aqueous solution (0.9 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in Vitamin E TPGS (171 mg) and cooled to room temperature.

### Example 70

Compound A (45 mg) was dissolved by heating in purified water (0.9 mL) and then cooled to room temperature. An aqueous solution (0.9 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in Gelucire 44/14 (171 mg) and cooled to room temperature.

### Example 71

Compound A (45 mg) was dissolved by heating in purified water (0.9 mL) and then cooled to room temperature. An aqueous solution (0.9 mL) prepared by dissolving sodium salicylate in an amount equimolar with Compound A was added to this solution, sufficiently stirred and centrifuged (15000 rpm, 10 minutes) to remove a supernatant, whereby precipitates were obtained. The precipitates were dried under reduced pressure, dissolved by heating in a solution of LABRASOL/LABRAFIL WL 2609 BS (9/8) (171 mg) and cooled to room temperature.

### Example 72

Sodium deoxycholate (160 mg) was added to Compound A (200 mg), mixed and milled for 3 hours. A solution (946 mg) prepared by mixing LABRASOL, LABRAFIL WL 2609 BS and TRANSCUTOL in the ratio of 45 : 40 : 15 was mixed with the mixture (54 mg) and stirred sufficiently under heating to prepare a uniform dispersion liquid of fine particles.

### Example 73

Sodium deoxycholate (480 mg) was added to Compound A (200 mg), mixed and milled for 3 hours. A solution (898 mg) prepared by mixing LABRASOL, LABRAFIL WL 2609 BS and TRANSCUTOL in the ratio of 45 : 40 : 15 was added to the mixture (102 mg) and stirred sufficiently under heating to prepare a uniform dispersion liquid of fine particles.

### Example 74

Sodium salicylate (240 mg) was added to Compound A (797 mg), mixed and milled for 3 hours. A solution (960.8 mg) prepared by mixing LABRASOL, LABRAFIL WL 2609 BS and TRANSCUTOL in the ratio of 45 : 40 : 15 was added to the mixture (39.2 mg) and stirred sufficiently under heating to prepare a uniform dispersion liquid of fine particles.

### Example 75

Sodium salicylate (480 mg) was added to Compound A (531 mg), mixed and milled for 3 hours. A solution (942.7 mg) prepared by mixing LABRASOL, LABRAFIL WL 2609 BS and TRANSCUTOL in the ratio of 45 : 40 : 15 was added to the mixture (57.3 mg) and stirred sufficiently under heating to prepare a uniform dispersion liquid of fine particles.

### Example 76

A solution (38800 mg) prepared by mixing LABRASOL, LABRAFIL WL 2609 BS and TRANSCUTOL in the ratio of 45 : 40 : 15 was added to Compound A (1200 mg). The mixture was treated with a galling homogenizer (Micron Lab) to prepare a uniform dispersion liquid of fine particles.

### Example 77

Sodium deoxycholate (800 mg) was added to Compound A (1000 mg), mixed and milled for 3 hours. A solution (1100 mg) prepared by mixing LABRASOL, LABRAFIL WL 2609 BS and TRANSCUTOL in the ratio of 45 : 40 : 15 was added to the mixture (900 mg) and stirred sufficiently under heating to prepare a uniform dispersion liquid of fine particles.

### Example 78

The preparation prepared in Example 77 was charged into HPMC capsules to prepare capsules.

### Experimental Example 2

The capsules prepared in Example 78 were orally administered to beagles which had previously been fasted for 24 hours. 0.5, 1, 2, 4, 6 and 8 hours after the administration, blood was collected via foreleg veins from the beagles. The concentration of Compound A in the plasma samples obtained by centrifugation were measured, and AUC for 8 hours after the administration was determined by a trapezoidalmethod. As the control, a suspension containing Compound A was similarly orally administered, and AUC for 8 hours after the administration was similarly determined.

As a result, the absorption was significantly increased by administering the capsules than by administering the suspension, as shown in Table 2.

**Table 2**

| Preparations | AUC (µg × hr/mL) |
|---|---|
| Control (suspension) | 0.191 |
| Example 78 | 0.795 |

### Example 79

Compound A (400 mg) was dissolved in a mixed solution of purified water/acetone (3/7). Sodium dodecyl sulfate in an amount equimolar with Compound A was added thereto and dissolved. Under stirring, the resultant mixture of purified water/acetone (3/7) was mixed with an equal volume of hexane. The mixture was subjected to vacuum suction and washed with purified water to give precipitates which were then dried under reduced pressure, to give white powders. The resultant white crystals were analyzed by NMR for elemental analysis and by MS mass spectrometry, to confirm that the product was dodecyl sulfate of Compound A.

### Example 80

A solution (4760 mg) prepared by mixing LABRASOL, LABRAFIL WL 2609 BS and TRANSCUTOL in the ratio of 45 : 40 : 15 was added to the dodecyl sulfate of Compound A (240 mg) obtained in Example 79 and stirred sufficiently under heating to prepare a uniform dispersion liquid of fine particles.

### Example 81

A solution (4760 mg) of Gelucire 44/14 melted at a temperature higher by at least 20 °C than the melting point thereof was added to the dodecyl sulfate of Compound A (240 mg) obtained in Example 79 and sufficiently stirred to prepare a uniform dispersion liquid of fine particles.

### Example 82

Compound A (400 mg) was dissolved at 40 wt% in lactic acid heated at 80 °C. A solution of Sefsol-228 (2250 mg), egg lecithin (45 mg), Cremophor EL (495 mg) uniformly mixed at 50 °C was added to this solution (210 mg), and sufficiently stirred to prepare a transparent solution.

### INDUSTRIAL APPLICABILITY

The pharmaceutical composition for oral administration according to the present invention is a preparation for oral administration superior in bioavailability with very high oral absorbability, thus showing little scatter in the concentration of the CCR5 antagonist in blood, and can be used for preventing or treating various HIV infections in humans, for example prevention and treatment of AIDS.

## Claims

1. A pharmaceutical composition for oral use, which comprises a water-insoluble or slightly soluble compound having CCR5 antagonism or a salt thereof dissolved or dispersed in a carrier comprising at least one component selected from amphipathic substances, alcohols and lipids.

2. A pharmaceutical composition for oral use, which comprises a water-insoluble or slightly soluble compound having CCR5 antagonism or a salt thereofdissolved in a carrier comprising at least one component selected from amphipathic substances, alcohols and lipids.

3. A pharmaceutical composition for oral use, which comprises a water-insoluble or slightly soluble compound having CCR5 antagonism or a salt thereof dispersed in a carrier comprising at least one component selected from amphipathic substances, alcohols and lipids.

4. The composition according to any one of claims 1 to 3, which is a liquid at ordinary temperature.

5. The composition according to any one of claims 1 to 3, which is a semisolid at ordinary temperature.

6. The composition according to any one of claims 1 to 3, which is a solid at ordinary temperature.

7. The composition according to any one of claims 1 to 3, wherein the carrier comprises an amphipathic substance.

8. The composition according to any one of claims 1 to 3, wherein the carrier comprises at least one amphipathic substance having high HLB and at least one amphipathic substance having low HLB respectively.

9. The composition according to claim 8, wherein the carrier comprises at least one kind of alcohol.

10. The composition according to any one of claims 1 to 3, wherein the carrier comprises an alcohol.

11. The composition according to any one of claims 1 to 3, wherein the carrier comprises a lipid.

12. The composition according to any one of claims 1 to 3, wherein the carrier comprises at least one alcohol and at least one lipid respectively.

13. The composition according to any one of claims 1 to 3, wherein the carrier comprises at least one alcohol and at least one amphipathic substance respectively.

14. The composition according to any one of claims 1 to 3, wherein the carrier comprises at least one lipid and at least one amphipathic substance respectively.

15. The composition according to any one of claims 1 to 3, wherein the carrier comprises at least one amphipathic substance, at least one alcohol and at least one lipid respectively.

16. The composition according to any one of claims 1 to 3, wherein the carrier comprises an absorption-promoting additive.

17. The composition according to claim 16, wherein the absorption-promoting additive is an alkali metal salt or alkaline earth metal salt with an organic acid.

18. The composition according to claim 17, wherein the organic acid is salicylic acid, deoxycholic acid, myristic acid, dodecylsulfuric acid, decylsulfuric acid, tetradecylsulfuric acid or lauric acid.

19. The composition according to claim 16, wherein the absorption-promoting additive is sodium salicylate, sodium deoxycholate, sodium myristate, sodium dodecylsulfate, sodium decylsulfate, sodium tetradecylsulfate or sodium laurate.

20. The composition according to any one of claims 1 to 3, wherein the amphipathic substance is a self-emulsifiable surfactant.

21. The composition according to claim 7, wherein the amphipathic substance is a combination of one or more amphipathic substances selected from saturated polyglycolized glycerides, unsaturated polyglycolized glycerides and D-α-tocopheryl polyethylene glycol 1000 succinate.

22. The composition according to claim 21, which further comprises an alkali metal salt or alkaline earth metal salt with an organic acid.

23. The composition according to claim 10, wherein the alcohol is propylene glycol, lauryl alcohol or diethylene glycol monoethyl ether.

24. A pharmaceutical composition for oral use, wherein an emulsion is formed by dissolving or dispersing a water-insoluble or slightly soluble compound having CCR5 antagonism or a salt thereof in a carrier comprising at least one component selected from amphipathic substances, alcohols and lipids, followed by adding water thereto.

25. The composition according to any one of claims 1 to 3, wherein the compound having CCR5 antagonism or a salt thereof is a compound represented by formula (I):
**R**^{**1**}**―X**^{**1**}**―W―X**^{**2**}**―Z**^{**1**}**―Z**^{**2**}**―R**^{**2**}
wherein R¹ represents an optionally substituted 5- to 6-membered ring, X¹ represents a bond or a divalent group having 1 to 4 atoms in the linear chain moiety thereof, W is a divalent group represented by the following formula: 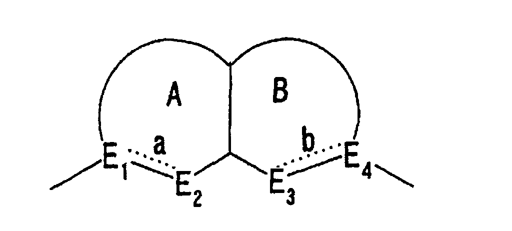 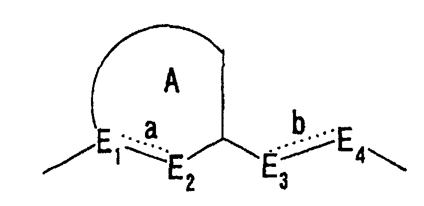 or 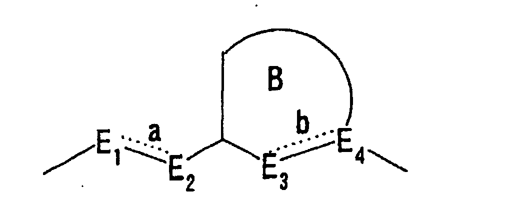 wherein rings A and rings B each represent an optionally substituted 5- to 7-membered ring, E₁ and E₄ each represent an optionally substituted carbon atom or an optionally substituted nitrogen atom, E₂ and E₃ each represent an optionally substituted carbon atom, an optionally substituted nitrogen atom, an optionally oxidized sulfur atom or an oxygen atom, and break lines a and b each represent a single or double bond; X² represents a divalent group having 1 to 4 atoms in the linear-chain moiety thereof, Z¹ represents a bond or a divalent cyclic group, Z² represents a bond or a divalent group having 1 to 4 carbon atoms in the linear-chain moiety thereof, R² represents (1) optionally substituted amino group whose nitrogen atom may be converted into quaternary ammonium or oxide, (2) optionally substituted nitrogen-containing heterocyclic group whose nitrogen atom may be converted into quaternary ammonium or oxide and which may contain a sulfur atom or oxygen atom as a ring constituent atom, (3) group bound via a sulfur atom, (4) group represented by the following formula: 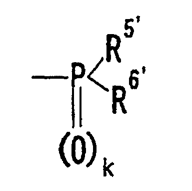 wherein k is 0 or 1, and when k is 0, the phosphorus atom may form a phosphonium salt, and R⁵' and R⁶' each represent an optionally substituted hydrocarbon group, an optionally substituted hydroxy group or an optionally substituted amino group, and R⁵' and R⁶' may be bound to each other to form a cyclic ring with their adjacent phosphorus atom, (5) optionally substituted amidino group, or (6) optionally substituted guanidino group, or a salt thereof.

26. The composition according to any one of claims 1 to 3, wherein the compound having CCR5 antagonism or a salt thereof is a compound represented by formula (Ia): 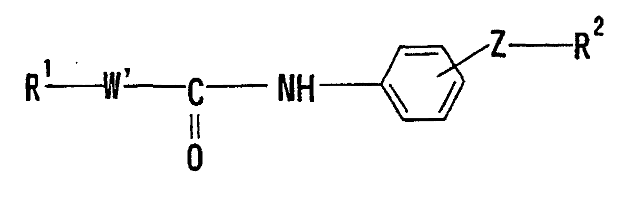 wherein R¹ has the same meaning as defined in claim 25, and W' is a divalent group represented by: 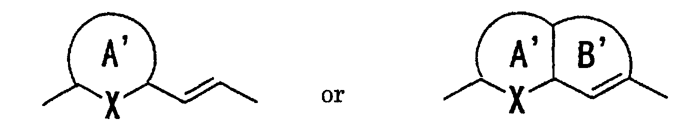 wherein ring A' represents an optionally substituted 5- to 6-membered aromatic ring, X represents an optionally substituted a carbon atom, an optionally substituted a nitrogen atom, a sulfur atom or an oxygen atom, and ring B' represents an optionally substituted 5- to 7-membered ring; Z represents a bond or a divalent group having 1 to 4 carbon atoms in the linear-chain moiety thereof, and R² has the same meaning as defined in claim 25, or a salt thereof.

27. The composition according to any one of claims 1 to 3, wherein the compound having CCR5 antagonism or a salt thereof is N,N-dimethyl-N-(4-(((2-(4-methylphenyl)-6,7-dihydro-5H-benzocyclohepten-8-yl)carbonyl)amino)benzyl)-N-(4-tetrahydropyranyl)ammonium chloride.

28. The composition according to any one of claims 1 to 3, wherein the compound having CCR5 antagonism or a salt thereof is a salt with an organic acid.

29. The composition according to claim 28, wherein the organic acid is salicylic acid, deoxycholic acid, myristic acid, dodecylsulfuric acid, decylsulfuric acid, tetradecylsulfuric acid or lauric acid.

30. The composition according to any one of claims 1 to 3, which is an anti-HIV drug.

31. A salt of a water-insoluble or slightly soluble compound having CCR5 antagonism formed with salicylic acid, deoxycholic acid, myristic acid, dodecylsulfuric acid, decylsulfuric acid, tetradecylsulfuric acid or lauric acid.

32. A salt comprising N,N-dimethyl-N-(4-(((2-(4-methylphenyl)-6,7-dihydro-5H-benzocyclohepten-8-yl) carbonyl)amino)benzyl)-N-(4-tetrahydropyranyl)ammonium and an anion derived from salicylic acid, deoxycholic acid, myristic acid, dodecylsulfuric acid, decylsulfuric acid, tetradecylsulfuric acid or lauric acid.
